(19) Europäisches Patentamt
European Patent Office
Office européen des brevets

(11) **EP 3 838 132 B1**

(12) **EUROPÄISCHE PATENTSCHRIFT**

(45) Veröffentlichungstag und Bekanntmachung des Hinweises auf die Patenterteilung:
**24.01.2024   Patentblatt 2024/04**

(21) Anmeldenummer: **19218075.0**

(22) Anmeldetag: **19.12.2019**

(51) Internationale Patentklassifikation (IPC):
*A61B 5/30* (2021.01)          *A61B 5/276* (2021.01)
*A61B 5/369* (2021.01)          *A61B 5/389* (2021.01)

(52) Gemeinsame Patentklassifikation (CPC):
**A61B 5/7221; A61B 5/276; A61B 5/30;**
**A61B 5/7203; A61B 5/7225;** A61B 5/369;
A61B 5/389; A61B 2562/222

(54) **UNTERDRÜCKUNG VON ECHOEFFEKTEN AUF ELEKTRODEN BEI DER MESSUNG VON BIOELEKTRISCHEN SIGNALEN**

SUPPRESSION OF ECHO EFFECTS ON ELECTRODES IN THE MEASUREMENT OF BIOELECTRICAL SIGNALS

SUPPRESSION DES EFFETS ÉCHO SUR LES ÉLECTRODES LORS DE LA MESURE DES SIGNAUX BIOÉLECTRIQUES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**23.06.2021   Patentblatt 2021/25**

(73) Patentinhaber: **Siemens Healthcare GmbH**
**91052 Erlangen (DE)**

(72) Erfinder:
• **Batzer, Ulrich**
**91080 Spardorf (DE)**

• **Brumhard, Matthias**
**91054 Erlangen (DE)**

(74) Vertreter: **Siemens Healthineers**
**Patent Attorneys**
**Siemens Healthcare GmbH**
**SHS TE IP**
**Henkestraße 127**
**91052 Erlangen (DE)**

(56) Entgegenhaltungen:
EP-A1- 3 569 143          DE-A1-102015 202 447
DE-A1-102017 214 862     US-A1- 2008 312 523
US-A1- 2011 001 497       US-A1- 2018 249 960

**Beschreibung**

Die Erfindung betrifft eine Störsignalmesseinrichtung in einem differentiellen Spannungsmesssystem mit einer Signalmessschaltung zur Messung von bioelektrischen Signalen mit einer Anzahl von Nutzsignalpfaden mit jeweils einer Sensorelektrode. Weiterhin betrifft die Erfindung eine Störsignalkompensationseinrichtung. Überdies betrifft die Erfindung ein differentielles Spannungsmesssystem. Außerdem betrifft die Erfindung ein Verfahren zur Erzeugung eines störreduzierten biologischen Messsignals.

Spannungsmesssysteme, insbesondere differentielle Spannungsmesssysteme, zur Messung von bioelektrischen Signalen werden beispielsweise in der Medizin zur Messung von Elektrokardiogrammen (EKG), Elektroenzephalogrammen (EEG) oder Elektromyogrammen (EMG) genutzt. Bei solchen Anwendungen sollte vorzugsweise auf jedem Messkanal auf eine hohe Eingangsimpedanz von mindestens mehreren MOhm geachtet werden, um den Einfluss von Störungen zu reduzieren oder zumindest nicht zu verstärken. Die hohe angestrebte Eingangsimpedanz sollte auch in den Kabeln der oben genannten Geräte zur Messung von bioelektrischen Signalen gehalten werden. Üblicherweise sind die Messleitungen der Kabel von einer Schirmung umgeben. Um eine bessere Handhabbarkeit zu erlangen, sind die Kabel zudem flexibel, schlank und leicht. Diese Merkmale führen jedoch zu einem Konflikt zwischen der Lebensdauer und der Handhabbarkeit der Kabel und erhöhen das Risiko eines Kabeldefekts.

Eine Gruppe von Störungen, die bei Messungen von bioelektrischen Signalen auftreten können, sind Störsignale, die beispielsweise am Patienten oder in die Kabel oder die Elektroden einkoppeln bzw. daran anliegen. Zu diesen Störsignalen zählen z.B. elektromagnetische Felder der Umgebung, elektrostatische Aufladungen und dergleichen.

Bei einer Einkopplung eines elektrischen Felds auf die Elektroden fließt der einkoppelnde Strom über den Körper und weiter über die EKG-Komponente auf Erde ab und verursacht bei dem Übergang über die Kontaktelektrode eine Störspannung.

In US 7,993,167 B2 wird ein Verfahren beschrieben, bei dem durch eine Schirmung Störsignale, welche durch Einkopplung elektrischer Felder auf Elektroden entstehen, vermieden werden sollen. Allerdings hat eine solche Schirmung den Nachteil, dass sie nur einen Teil der Einkopplungen vermeiden kann, da eine solche Schirmung an mehreren Stellen von der Haut eines Patienten absteht, so dass zwischen Körper und Elektrodenschirmung Störungen einkoppeln können.

In US 2008 / 312523 A1 wird eine Messvorrichtung umfassend eine Vielzahl von Messleitungen und Messelektroden sowie eine Vielzahl von Referenzleitungen beschrieben, mittels welcher Interferenzen unterdrückt werden können. Dazu werden Referenzsignale von den eigentlichen Messsignalen subtrahiert.

In DE 10 2017 214 862 A1 und EP 3 569 143 A1 wird jeweils eine Fehlerdetektionsvorrichtung beschrieben, die Signalpfaddefekte wie bspw. Kinken detektiert.

In DE 10 2015 202 447 A1 wird ein differentielles Spannungsmesssystem beschrieben, bei dem durch Gleichtaktsignale gestörte bioelektrische Messsignale zu korrigieren.

Es ist daher eine Aufgabe der vorliegenden Erfindung, auch Störungen bei der Messung bioelektrischer Signale, die auf die Elektroden einwirken, zu verringern oder sogar zu vermeiden.

Diese Aufgabe wird durch eine Störsignalmesseinrichtung gemäß Patentanspruch 1, durch eine Störsignalkompensationseinrichtung gemäß Patentanspruch 3, durch ein differentielles Spannungsmesssystem gemäß Patentanspruch 4 sowie durch ein Verfahren zur Erzeugung eines störreduzierten biologischen Messsignals gemäß Patentanspruch 12 gelöst.

Die erfindungsgemäße Störsignalmesseinrichtung dient zur Messung von Störsignalen auf Nutzsignalpfaden in einem differentiellen Spannungsmesssystem mit einer Signalmessschaltung zur Messung von bioelektrischen Signalen mit einer Anzahl von Nutzsignalpfaden mit jeweils einer Sensorelektrode. Die Störsignalmesseinrichtung umfasst eine zusätzliche Sensorleitung pro Sensorelektrode, welche mit einer Masseverbindung einer Zuleitung einer Sensorelektrode elektrisch verbunden ist. Teil der Störsignalmesseinrichtung ist auch eine Messverstärkerschaltung je Sensorelektrode, welche über einen elektrischen Widerstand mit der zusätzlichen Sensorleitung verbunden ist und dazu eingerichtet ist, eine auf der Sensorleitung auftretende elektrische Potentialänderung zu erfassen und daraus ein Elektroden-Referenz-Störsignal zu ermitteln.

Das differentielle Spannungsmesssystem erfasst, wie eingangs bereits erwähnt, bioelektrische Signale z. B. von einem menschlichen oder tierischen Patienten. Dazu weist es eine Anzahl von Messleitungen bzw. Nutzsignalpfaden auf. Diese verbinden zum Beispiel als einzelne Kabel Elektroden, die am Patienten zur Erfassung der Signale angebracht sind, mit den weiteren Komponenten des Spannungsmesssystems, also insbesondere der Elektronik, die zur Auswertung bzw. Darstellung der erfassten Signale dient.

Differenzielle Spannungsmesssysteme sind dem Fachmann ihrer grundlegenden Funktionsweise nach bekannt, weswegen an dieser Stelle auf eine detailliertere Erläuterung verzichtet wird. Sie können insbesondere als Elektrokardiogramme (EKG), Elektroenzephalogramme (EEG) oder Elektromyogramme (EMG) ausgebildet sein.

Als Störungen werden dabei sowohl Störsignale, wie z. B. Einkopplungen in die Kabel oder über einen Patienten, als auch Signalpfaddefekte, wie z. B. Kabelbrüche, Kinken oder dergleichen bezeichnet. Einkopplungen treten bei der Messung von bioelektrischen Signalen, beispielsweise von EKG-Signalen, z. B. häufig als Gleichtaktstörsignale, auch "Common Mode"-Signale (CM-Signale) genannt, auf.

Erfindungsgemäß werden nun Störsignale, welche auf die Sensorelektroden des differentiellen Spannungsmesssystems eingekoppelt werden, durch eine gesonderte Störsignalmesseinrichtung erfasst. Vorteilhafterweise können damit Störsignale von Störquellen, wie zum Beispiel einem stromführenden Kabel, welches vor allem ein Störsignal auf einer Elektrode einkoppelt, aber kaum Störsignale auf den Körper des Untersuchungsobjekts einkoppelt, gesondert gemessen werden. Durch die separate Analyse können überdies konkrete Handlungsanweisungen für den Nutzer gegeben werden, durch die die Störquelle entfernt werden kann. Außerdem kann das gemessene Störsignal auch als Elektroden-Referenz-Störsignal zur gezielten Unterdrückung eines Störanteils in dem Messsignal verwendet werden. Hierzu können beispielsweise mehrere adaptive Filter genutzt werden, mit denen unterschiedliche Störanteile aus dem Messsignal herausgefiltert werden.

Die erfindungsgemäße Störsignalkompensationseinrichtung weist die erfindungsgemäße Störsignalmesseinrichtung auf. Weiterhin umfasst die erfindungsgemäße Störsignalkompensationseinrichtung eine Auswerteeinheit. Die Auswerteeinheit umfasst eine erste adaptive Filtereinheit, welche dazu eingerichtet ist, ein erstes störreduziertes Messsignal auf Basis eines Messsignals und eines Referenz-Gleichtaktstörsignals zu erzeugen. Ein solches Referenz-Gleichtaktstörsignal umfasst Störsignale, welche auf den Körper des Untersuchungsobjekts eingekoppelt sind. Eine adaptive Filtereinheit besitzt die Eigenschaft, ihre Übertragungsfunktion und seine Frequenz im Betrieb selbständig ändern zu können. Dabei wird in Abhängigkeit von einem Ausgangssignal des Filters ein Fehlersignal erzeugt, und die Filterkoeffizienten werden in Abhängigkeit von dem Fehlersignal derart geändert, dass das Fehlersignal minimiert wird. Das erste störreduzierte Messsignal enthält vor allem weniger Störsignale, welche von Störungen herrühren, die auf den Körper des Untersuchungsobjekts eingekoppelt wurden. Allerdings reduziert die erste adaptive Filtereinheit, wenn auch in geringerem Maße, auch Störsignale, welche durch eine Störung verursacht wurden, die auf die Elektroden einkoppelt. Mithin passt das von der Störsignalmesseinrichtung erfasste Elektroden-Referenz-Störsignal nicht mehr gut als Referenz für das durch die erste Filtereinheit erzeugte erste störreduzierte Messsignal.

Dieser Effekt erklärt sich dadurch, dass jedes elektrische Feld in der Nähe des Patienten immer gleichzeitig auf Patient und Elektrode einkoppelt. Je nach Position und Art der Störung erfolgt dies sehr unterschiedlich stark. Da die Quelle der Störung für Elektroden- und Referenzstörung das gleiche elektrische Feld ist, enthält die Gleichtakt-Referenzmessung je nach Einkopplung zumindest Anteile der Störung auf die Elektrode. Die erste adaptive Filtereinheit versucht nun diese gleichen Anteile zu entfernen und entfernt dadurch nicht nur die Gleichtakt-Störanteile sondern auch teilweise die Einkopplung auf die Elektroden.

Aus diesem Grund umfasst die Auswerteeinheit eine zweite adaptive Filtereinheit, mit der ein angepasstes Referenz-Elektroden-Störsignal erzeugt wird, welches nur noch Störanteile enthält, die durch die erste adaptive Filtereinheit nicht herausgefiltert wurden. Die zweite adaptive Filtereinheit, welche vorzugsweise parallel zu der ersten adaptiven Filtereinheit geschaltet ist, ist dazu eingerichtet, ein um Gleichtaktstörungen reduziertes angepasstes Referenz-Elektroden-Störsignal auf Basis des von der Störsignalmesseinrichtung ermittelten Elektroden-Referenz-Störsignals und des Referenz-Gleichtaktstörsignals zu erzeugen. Wie bereits erwähnt, werden dabei auch Störanteile, welche durch eine Einkopplung von Störfeldern auf die Elektroden herrühren, aber auch in dem Referenz-Gleichtaktstörsignal enthalten sind, herausgefiltert. Wie bereits erwähnt, wird auf diese Weise ein angepasstes Referenz-Elektroden-Störsignal, welches nur noch Störsignalanteile enthält, die durch die erste adaptive Filtereinheit nicht herausgefiltert wurden. Dieses angepasste Referenz-Elektroden-Störsignal kann nun als Referenzsignal von einer dritten adaptiven Filtereinheit genutzt werden, die ebenfalls Teil der Auswerteeinheit ist. Die dritte adaptive Filtereinheit ist der ersten und der zweiten adaptiven Filtereinheit nachgeschaltet und ist dazu eingerichtet, ein zweites störreduziertes Messsignal auf Basis des ersten störreduzierten Messsignals und des angepassten Referenz-Elektroden-Störsignals zu ermitteln.

Dieses zweite störreduzierte Messsignal ist nun vorteilhaft effektiv sowohl von Störsignalen befreit, die durch Einkopplung von Störfeldern auf den Körper des Untersuchungsobjekts erzeugt wurden, als auch von Störsignalen befreit, die von Einkopplungen von Störfeldern auf die Sensorelektroden des differentiellen Spannungsmesssystems erzeugt wurden.

Das erfindungsgemäße differentielle Spannungsmesssystem weist mindestens eine erste Elektrode und eine zweite Elektrode zur Messung von bioelektrischen Messsignalen auf. Weiterhin weist das erfindungsgemäße differentielle Spannungsmesssystem mindestens eine dritte Elektrode für einen Potentialausgleich zwischen einem Messobjekt und dem differentiellen Spannungsmesssystem auf. Mittels dieser dritten Elektrode kann auch das bereits erwähnte Referenz-Gleichtaktstörsignal erzeugt werden. Zudem weist das erfindungsgemäße differentielle Spannungsmesssystem eine Messeinrichtung auf. Die Messeinrichtung weist eine Signalmessschaltung zur Messung der bioelektrischen Signale auf. Weiterhin weist die Messeinrichtung auch eine Referenzsignaleinheit auf, welche das erwähnte Referenz-Gleichtaktstörsignal erzeugt und hierzu sowohl mit der bereits erwähnten dritten Elektrode als auch mit der Signalmessschaltung verbunden ist.

Zur Messung des Referenz-Gleichtaktstörsignals weist das differentielle Spannungsmesssystem einen dritten Nutzsignalpfad mit der bereits erwähnten dritten Elektrode auf. Des Weiteren umfasst das differentielle Spannungsmesssystem vorzugsweise eine Treiberschaltung, welche zwischen einem Strommesswiderstand und die Signalmessschaltung geschaltet ist. Die Treiberschaltung wird auch als "Right-Leg-Drive" (RLD) bezeichnet und ist für die Erzeugung eines Signals verantwortlich, welches auf die Durchschnitts-Gleichtaktspannung einzelner oder aller Signale geregelt wird. Die bereits oben erwähnten und gemessenen Gleichtaktstörsignale, können dadurch in den Nutzsignalpfaden reduziert werden.

Der dritte Nutzsignalpfad (bzw. "Right-Leg-Drive-Pfad") sorgt für einen Potentialausgleich zwischen dem Patienten und dem differentiellen Spannungsmesssystem bzw. dem EKG-Messsystem. Dabei ist die Elektrode des dritten Nutzsignalpfads vorzugsweise auf dem rechten Bein des Patienten angebracht, worauf die Bezeichnung "Right-Leg-Drive" zurückzuführen ist. Grundsätzlich kann dieses dritte Potential aber auch an anderer Stelle am Patienten erfasst werden.

Außerdem weist das erfindungsgemäße differentielle Spannungsmesssystem auch die erfindungsgemäße Störsignalmesseinrichtung auf. Das erfindungsgemäße differentielle Spannungsmesssystem teilt die Vorteile der erfindungsgemäßen Störsignalkompensationseinrichtung und der erfindungsgemäßen Störsignalmesseinrichtung.

Bei dem erfindungsgemäßen Verfahren zur Erzeugung eines störreduzierten biologischen Messsignals, wird ein möglicherweise störbehaftetes Messsignal erfasst. Zudem wird ein Elektroden-Referenz-Störsignal über eine Messverstärkerschaltung je Sensorelektrode erfasst. Die jeweilige Messverstärkerschaltung ist über einen elektrischen Widerstand mit der zusätzlichen Sensorleitung verbunden und ist dazu eingerichtet, eine auf der Sensorleitung auftretende elektrische Potentialänderung zu erfassen und daraus ein Elektroden-Referenz-Störsignal zu ermitteln. Außerdem wird ein Referenz-Gleichtakt-Störsignal erfasst. Auf Basis des Messsignals und des Referenz-Gleichtaktstörsignals wird ein erstes störreduziertes Messsignal ermittelt. Weiterhin wird ein um Gleichtaktstörungen reduziertes angepasstes Referenz-Elektroden-Störsignal auf Basis des von der Störsignalmesseinrichtung ermittelten Elektroden-Referenz-Störsignals und des Referenz-Gleichtaktstörsignals ermittelt. Schließlich wird ein zweites störreduziertes Messsignal auf Basis des ersten störreduzierten Messsignals und des angepassten Referenz-Elektroden-Störsignals ermittelt. Das erfindungsgemäße Verfahren zur Erzeugung eines störreduzierten biologischen Messsignals teilt die Vorteile der erfindungsgemäßen Störsignalkompensationseinrichtung und der erfindungsgemäßen Störsignalmesseinrichtung.

Ein Großteil der zuvor genannten Komponenten der erfindungsgemäßen Störsignalkompensationseinrichtung, insbesondere die Auswerteeinheit, kann ganz oder teilweise in Form von Softwaremodulen in einem Prozessor eines entsprechenden Spannungsmesssystems realisiert werden. Eine weitgehend softwaremäßige Realisierung hat den Vorteil, dass auch schon bisher verwendete Spannungsmesssysteme auf einfache Weise bei Ergänzung nötiger Hardware-Komponenten, wie zum Beispiel der zusätzlichen Sensorleitungen und Messverstärkerschaltungen, durch ein Software-Update nachgerüstet werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung eines Spannungsmesssystems ladbar ist, mit Programmabschnitten, um alle Schritte des erfindungsgemäßen Verfahrens auszuführen, wenn das Programm in dem Spannungsmesssystem ausgeführt wird. Ein solches Computerprogrammprodukt kann neben dem Computerprogramm gegebenenfalls zusätzliche Bestandteile wie z. B. eine Dokumentation und/oder zusätzliche Komponenten auch Hardware-Komponenten, wie z.B. Hardware-Schlüssel (Dongles etc.) zur Nutzung der Software, umfassen.

Zum Transport zum Spannungsmesssystem und/oder zur Speicherung an oder in dem Spannungsmesssystem kann ein computerlesbares Medium, beispielsweise ein Memorystick, eine Festplatte oder ein sonstiger transportabler oder fest eingebauter Datenträger dienen, auf welchem die von einer Rechnereinheit des Spannungsmesssystems einlesbaren und ausführbaren Programmabschnitte des Computerprogramms gespeichert sind. Die Rechnereinheit kann z.B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung, wobei die Ansprüche einer Anspruchskategorie auch analog zu den Ansprüchen und Beschreibungsteilen zu einer anderen Anspruchskategorie weitergebildet sein können und insbesondere auch einzelne Merkmale verschiedener Ausführungsbeispiele bzw. Varianten zu neuen Ausführungsbeispielen bzw. Varianten kombiniert werden können.

In einer Variante der erfindungsgemäßen Störsignalmesseinrichtung weist die Messverstärkerschaltung eine programmierbare Verstärkerschaltung auf. Mit einer solchen Schaltung lässt sich die Empfindlichkeit einer Messung mit einem AD-Wandler, der Signale nur diskret und von deren Betriebsspannung abhängig verarbeiten kann, verbessern. Je nach Typ und Hersteller kann die Rückkoppelung durch das Beschalten von einzelnen Pins (parallel), den SPI- oder I$^2$C-Bus (seriell) geändert und so die Verstärkung verstellt werden. Teilweise wird auch ein programmierbarer Analogmultiplexer verbaut, durch den zwischen den verschiedenen Eingangskanälen umgeschaltet werden kann.

Erfindungsgemäß ist die Messverstärkerschaltung an die Schirmung einer Zuleitung der Sensorelektrode elektrisch angeschlossen.

Die Nutzsignalpfade weisen zumindest eine Schirmung bzw. Abschirmung (engl. "shield") auf. Die Schirmung dient dazu, insbesondere bei höheren Frequenzen auftretende elektrische und/oder magnetische Felder von den Nutzsignalpfaden fernzuhalten. Sie wirkt dabei nach dem Prinzip Influenz bzw. wie ein faradayscher Käfig und ist beispielsweise als eine den Leiter des Nutzsignalpfads umgebende, jedoch von diesem isolierte metallische Folie oder dergleichen ausgebildet. Dabei kann jeder Nutzsignalpfad eine separate Schirmung in dem Sinne aufweisen, dass jede Schirmung an ein eigenes Referenzpotenzial bzw. an eine eigene Auswerteelektronik angeschlossen ist. Die Nutzsignalpfade weisen aber bevorzugt eine gemeinsame Schirmung in dem Sinne auf, dass Schirmungsteile, die die einzelnen Nutzsignalpfade umgeben, elektrisch leitend verbunden sind und an ein gemeinsames Referenzpotenzial bzw. eine gemeinsame Elektronik angeschlossen sind.

Die Schirmung kann dabei grundsätzlich passiv ausgebildet sein, indem sie mit dem Massepotential der Signalmessschaltung verbunden ist. Sie kann aber bevorzugt auch aktiv über einen entsprechenden Treiber angesteuert werden, um die Einflüsse etwaiger Störfelder auszugleichen.

Prinzipiell lassen sich auf der Schirmung dieselben Störsignale messen, die auch die Nutzsignalpfade beaufschlagen. Denn elektromagnetische Felder der Umgebung, die zumeist von dem Gleichtaktanteil der Netzfrequenz dominiert werden, koppeln ähnlich in die Schirmung wie in den Nutzsignalpfad ein. Daher sind insbesondere diese Anteile mit Vorteilen für die weitere Analyse der Störsignale selbst und deren Einfluss auf die eigentlich zu messenden bioelektrischen Signale an der Schirmung zu messen.

Mithin kann durch Kontaktierung der Schirmung das Elektroden-Referenz-Störsignal gewonnen werden, welches erfindungsgemäß zur Entstörung des Messsignals genutzt wird.

In einer Variante des erfindungsgemäßen differentiellen Spannungsmesssystems weist die Messeinrichtung eine Detektionsvorrichtung zur Detektion von Störungen auf Signalpfaden in dem differentiellen Spannungsmesssystem auf.

Die genannte Detektionsvorrichtung dient zur Detektion von Störungen auf Signalpfaden in dem differentiellen Spannungsmesssystem, die eine Schirmung aufweisen. Die Detektionsvorrichtung umfasst dabei zumindest eine Analyseeinheit. Letztere ist mit der Schirmung verbunden und ausgebildet, um eine Störung in einem Nutzsignalpfad des Spannungsmesssystems mittels eines im Störfall an der Schirmung gemessenen Signals zu detektieren.

Die Analyseeinheit ist ausgebildet, um eine Störung in einem Nutzsignalpfad des Spannungsmesssystems zu detektieren. Die Störung wird also erfindungsgemäß detektiert, indem ein - im Optimalfall nicht erwartetes - Signal, also z. B. ein bioelektrisches Signal und/oder ein CM-Signal auf der Schirmung gemessen und ggf. detaillierter analysiert wird. Die Analyseeinheit kann dabei grundsätzlich unterschiedlich ausgebildet sein. Die Detektionsvorrichtung kann genau eine, in anderen Ausbildungsformen aber auch mehrere Analyseeinheiten, wie beispielsweise eine Defektanalyseeinheit und eine Störsignalauswerteeinheit umfassen. Die Analyseeinheit kann bevorzugt einen Integrated Circuit aufweisen, besonders bevorzugt einen ASIC. Die Analyseeinheit kann aber auch vorzugsweise einen Mikrocontroller umfassen oder eine sonstige universelle Recheneinheit.

Anstatt nur die Nutzsignalpfade bzw. die zu prüfenden Kabel selber zu prüfen, wird also auf der Schirmung gemessen und überprüft, ob die Nutzsignalpfade defekt sind oder andere Störungen auf die Schirmung übergekoppelt haben. Im Gegensatz zu aus der Praxis bekannten Anwendungen müssen dafür die Nutzsignalpfade nicht mit dem Patienten verbunden sein. Es kann also zu einem beliebigen Zeitpunkt also z.B. im Vorfeld einer Untersuchung, aber auch während der Untersuchung getestet werden, ob alle Kabel funktionstüchtig sind und/oder ob starke elektromagnetische Felder in die Kabel einkoppeln.

Ein weiterer Vorteil der Nutzung einer Detektionsvorrichtung besteht darin, dass kein Techniker beziehungsweise Servicepersonal mehr benötigt wird, um einen Kabeldefekt zu detektieren. Die Messung auf der Schirmung kann während einer Nutzsignalmessung parallel und automatisch verlaufen und ein Kabeldefekt kann sofort sichtbar gemacht werden, beispielsweise auf einer Benutzerschnittstelle des Spannungsmesssystems. Daher kann der Signalpfaddefekt, beziehungsweise Kabeldefekt, sofort z. B. durch das Bedienpersonal selber entdeckt werden und das Kabel kann sofort getauscht und eine korrekte Messung durchgeführt werden. Dadurch sinkt also auch die Gefahr, dass mit unentdeckt beschädigten Kabeln weiter gemessen wird.

Zudem spielt die Art des Nutzsignals bei der Detektion einer Störung mittels der Schirmung keine Rolle. Daher kann die Detektionsvorrichtung für verschiedenste Spannungsmesssysteme, wie beispielsweise EKG-Messsysteme, EEG-Messsysteme oder EMG-Messsysteme Anwendung finden, ohne dafür speziell angepasst werden zu müssen. Hierdurch können ebenfalls enorme Entwicklungs- und Herstellungskosten eingespart werden.

Ferner benötigt die Detektionsvorrichtung keine externe Spannungsquelle für eine Detektion von Störungen, da alle relevanten stromführenden Teile in der Detektionsvorrichtung bzw. der Spannungsmesseinrichtung integriert sein können bzw. integriert sind. Dadurch kann der Testaufbau komplett passiv sein.

Die Detektionsvorrichtung zur Detektion von Störungen auf Signalpfaden in einem differentiellen Spannungsmesssystem mit einer Signalmessschaltung zur Messung von bioelektrischen Signalen mit einer Anzahl von Nutzsignalpfaden weist vorzugsweise eine Störsignalauswerteeinheit auf. Diese wiederum umfasst eine Störsignalermittlungseinheit, die so ausgebildet ist, dass sie ein Störsignal mit einer Frequenz im Bereich einer Netzfrequenz ermittelt. Die Störsignalauswerteeinheit umfasst ferner eine Störsignalunterdrückungseinheit, die so ausgebildet ist, dass sie Störsignalanteile in den bioelektrischen Signalen auf Basis des ermittelten Störsignals verringert.

Die hier beschriebene Detektionsvorrichtung kann auch als eigenständige Idee unabhängig von der zuvor beschriebenen Detektionsvorrichtung, also auch ohne die Nutzung der Schirmung zur Detektion von Störungen genutzt werden, wenn die Störsignale auf einem anderen Weg, z. B. einem separaten Störsignalpfad, erfasst werden. Besondere synergetische Effekte ergeben sich jedoch, wenn die erfindungsgemäße Ermittlung des Störsignals im Bereich der Netzfrequenz durch eine Messung an der Schirmung erfolgt. Denn die Schirmung stellt einen elektrischen Leiter dar, der die gleiche Länge wie die Nutzsignalpfade hat. Elektromagnetische Felder der Umgebung, die zumeist von dem Gleichtaktanteil der Netzfrequenz dominiert werden, koppeln daher ähnlich in die Schirmung wie in den Nutzsignalpfad ein. Daher sind insbesondere diese Anteile mit Vorteilen für die weitere Analyse der Störsignale selbst und deren Einfluss auf die eigentlich zu messenden bioelektrischen Signale an der Schirmung zu messen.

Die Funktionsweise eines differentiellen Spannungsmesssystems wurde oben bereits eingehend erläutert. Als Netzfrequenz wird die Frequenz der üblicherweise landesweit von Stromversorgern zur Verfügung gestellten Netzspannung oder zumindest die Frequenz der Spannung des Stromkreises bezeichnet, an den die Detektionsvorrichtung angeschlossen ist. Es hat sich herausgestellt, dass die Ermittlung von Störsignalen wesentlich vereinfacht werden kann, wenn man den Frequenzbereich der Störsignale eingrenzt.

Die Störsignalermittlungseinheit ermittelt erfindungsgemäß also nur Störsignale, insbesondere CM-Signale, die im Bereich der Netzfrequenz liegen. Da diese üblicherweise die größten Störsignalanteile darstellen, ergibt sich dadurch eine erhebliche Vereinfachung bei der Signalanalyse. Im Rahmen der Signalanalyse wird in dem Frequenzbereich um die Netzfrequenz z. B. mittels einer Frequenzanalyse nach dem stärksten Signal gesucht. Dieses stellt dann mit hoher Wahrscheinlichkeit die Netzstörung dar, da auf den Störsignalpfaden im Regelfall keine Nutzsignalanteile aufzufinden sind.

Das aufgefundene Signal wird dann im Folgenden zur Störsignalunterdrückung genutzt, wie später noch detaillierter erläutert wird. Dabei wird das Signal jedoch nicht wie bisher üblich in einem weiten Bereich verformt, was den diagnostischen Wert verringern würde, sondern lediglich präzise im Rahmen der üblichen Toleranzen bei der zuvor ermittelten Frequenz des Störsignals so verändert, dass die Einflüsse des Störsignals reduziert bzw. kompensiert werden.

Die Art des Nutzsignals spielt bei der Detektion einer Störung mittels der Schirmung und/oder auf Basis eines Bereichs um die Netzfrequenz keine Rolle. Daher kann die erfindungsgemäße Detektionsvorrichtung für verschiedenste Spannungsmesssysteme, wie beispielsweise EKG-Messsysteme, EEG-Messsysteme oder EMG-Messsysteme Anwendung finden, ohne dafür speziell angepasst werden zu müssen. Hierdurch können ebenfalls enorme Entwicklungs- und Herstellungskosten eingespart werden.

Ferner benötigt die Detektionsvorrichtung keine externe Spannungsquelle für eine Detektion von Störungen, da alle relevanten stromführenden Teile in der Detektionsvorrichtung bzw. der Spannungsmesseinrichtung integriert sein können bzw. integriert sind. Dadurch kann der Testaufbau komplett passiv sein.

Die Detektionsvorrichtung kann dabei jeweils eine eigenständige Komponente sein und beispielsweise als Nachrüstsatz in bereits bestehende EKGs, EEGs oder EMGs z. B. über Steckverbindungen, eingebaut bzw. vor- oder zwischengeschaltet werden, was später noch näher erläutert wird. Vorzugsweise ist die Detektionsvorrichtung jedoch bereits fest in einem erfindungsgemäßen Spannungsmesssystem integriert.

Die Detektionsvorrichtung umfasst bevorzugt zumindest eine Stromaufbringungseinheit, die so ausgebildet ist, dass sie ein Signal auf einen ersten Nutzsignalpfad aufprägt. Die Detektionsvorrichtung umfasst ferner vorzugsweise eine Defektanalyseeinheit als Analyseeinheit, die einen Signalpfaddefekt in einem Nutzsignalpfad des Spannungsmesssystems mittels des zuvor aufgeprägten, im Störfall an der Schirmung gemessenen Signals detektiert.

Dabei ist die Stromaufbringungseinheit bevorzugt so ausgebildet, dass sie vorzugsweise ein beliebiges aber definiertes erstes Signal auf einen ersten Nutzsignalpfad der Signalmessschaltung des Spannungsmesssystems aufprägt. Bei dem Signal kann es sich vorzugsweise um einen Strom handeln, der direkt oder indirekt aufgeprägt und gemessen werden kann. So kann der Strom bevorzugt über eine Stromquelle auf einen Nutzsignalpfad aufgeprägt werden. Der Strom kann aber auch besonders bevorzugt über einen Pull-up-Widerstand und/oder einen Pull-up-down-Widerstand indirekt auf dem Nutzsignalpad aufgeprägt bzw. geregelt werden. Der aufgeprägte Strom liegt dabei vorzugsweise im Nanoampere-Bereich, um beispielsweise gemessene bioelektrische Signale nicht zu verfälschen und damit eine Gefährdung eines Patienten ausgeschlossen wird.

Ist der Nutzsignalpfad beispielsweise mittels einer Elektrode mit einem Patienten verbunden und prägt die Stromaufbringungseinheit ein Signal bzw. einen Strom auf den ersten Nutzsignalpfad auf, so fließt dieser Strom über den Patienten und einen entsprechend ausgelegten Rückpfad, der mit einem Patienten verbunden ist, wieder ab.

Bei einem entsprechend ausgelegten Rückpfad für den auf dem Nutzsignalpfad aufgeprägten Strom handelt es sich vorzugsweise um einen niederohmigen Rückpfad auf ein gemeinsames Bezugspotential. Solch ein Rückpfad wird beispielsweise durch den ersten Störsignalpfad gebildet. Da die Nutzsignalpfade aufgrund ihrer hohen Eingangsimpedanzen keinen niederohmigen Rückflusspfad auf das gemeinsame Bezugspotential bilden (bei intakten Kabeln), kann das aufgeprägte Signal nur über den Störsignalpfad abfließen. Das hat zur Folge, dass sich nicht nur die oben beschriebenen Störsignale auf dem Störsignalpfad befinden, sondern auch das auf dem Nutzsignalpfad aufgeprägte Signal.

Ist ein Signalmesskabel bzw. Kabel eines Nutzsignalpfads defekt, so weist die Signalmessschaltung neben dem Störsignalpfad mindestens einen weiteren niederohmigen Rückpfad für den aufgeprägten Strom auf. Das aufgeprägte Signal geht dann über in die Schirmung und fließt nicht mehr über den Störsignalpfad ab, da die Schirmung den geringeren elektrischen Widerstand aufweist. Das Signal auf dem Störsignalpfad wird also bei einem Kabeldefekt nicht mehr um das auf dem Nutzsignalpfad aufgeprägte Signal erhöht, sondern das aufgeprägte Signal ist in diesem Fall auf der Schirmung zu messen.

Diese Messung kann unabhängig davon erfolgen, ob das jeweilige Kabel mit dem Patienten verbunden ist oder nicht. Der Kabeldefekt kann auf diese Art bereits vor, aber auch während einer Untersuchung festgestellt und entsprechende Gegenmaßnahmen, wie z. B. ein Kabelaustausch, vorgenommen werden.

Bei einem eingangs genannten Spannungsmesssystem kann die Signalmessschaltung in Abhängigkeit von ihrer Anwendung eine beliebige Anzahl an Nutzsignalpfaden bzw. Signalmesskabeln aufweisen. In der Regel weist eine Signalmessschaltung, beispielsweise ein EKG-Messsystem, zumindest zwei Nutzsignalpfade auf. Die Nutzsignalpfade umfassen vorzugsweise Elektroden, welche an einen zu untersuchenden Patienten angelegt werden können, um ein dort anliegendes elektrisches Potential zu messen. Der Aufbau der Elektroden kann von der genauen Art der Messung abhängen, z.B. ob es sich um eine EKG-Messung, eine EEG-Messung oder eine EMG-Messung handelt, und davon, wo genau am Patienten das Potential gemessen werden soll. Geeignete Elektroden für verschiedene Einsatzzwecke sind dem Fachmann bekannt. Der Ausgang der Elektroden ist, vorzugsweise über die genannten Signalmesskabel, bevorzugt mit einer Verstärkerschaltung verbunden. Besonders bevorzugt sind die Elektroden mit einem Differenzverstärker elektrisch verbunden. Dieser bildet aus den an seinen Eingängen gemessenen und von den Elektroden erfassten Signalen eine Differenz und verstärkt diese. Zudem weist die Signalmessschaltung eine Signalerfassungseinheit auf, die am Ausgang der Verstärkerschaltung geschaltet ist, um die verstärkten Signale oder z.B. die Potentiale zu erfassen und weiter zu nutzen und/oder aufzuzeichnen. Z. B. kann die Signalerfassungseinheit einen A/D-Wandler und weitere Komponenten aufweisen, um das digitale Signal weiter zu verarbeiten.

Die Stromaufbringungseinheit prägt bevorzugt jeweils verschiedene, d. h. einzigartige, Signale auf unterschiedliche Nutzsignalpfade auf. Dies ist besonders vorteilhaft, wenn nicht alle Nutzsignalpfade separate Schirmungen, sondern eine gemeinsame Schirmung aufweisen, wie im Folgenden näher erläutert wird.

Die Schirmung oder andere Störsignalpfade weisen in der Regel keine oder nur sehr geringe Gleichstrom-Anteile auf. Wird das Signal auf dem Nutzsignalpfad als Gleichstrom aufgeprägt, so lässt sich dieser Anteil sehr leicht von häufiger eingekoppelten oder sonst vorhandenen Wechselstromanteilen unterscheiden.

Andernfalls kann es aber auch von Vorteil sein, das Signal als einen Wechselstrom auf den Nutzsignalpfad aufzuprägen.

Daher ist die Stromaufbringungseinheit bzw. Stromaufbringungskontrolleinheit vorzugsweise so ausgebildet, dass sie einen Wechselstrom und/oder einen Gleichstrom auf die Nutzsignalpfade aufprägen kann.

Vorzugsweise ist die Stromaufbringungseinheit so ausgebildet, dass die aufgeprägten Signale auf die Nutzsignalpfade positive Ströme umfassen. Dabei wird auf die Nutzsignalpfade der Signalmessschaltung jeweils ein positiver Strom aufgeprägt.

Somit ergibt sich bei einer Anzahl von N Nutzsignalpfaden ein Gesamtsignal $I_g$, bzw. Gesamtstrom $I_g$ von:

$$I_g = I_1 + I_2 + \cdots + I_N$$

Liegt kein Signalpfaddefekt vor, so fließt also zusätzlich zu den ggf. anliegenden Störsignalen auch der durch die Stromaufbringungseinheiten aufgeprägte Gesamtstrom über den ersten Störsignalpfad ab.

Weist die Signalmessschaltung mehrere Nutzsignalpfade auf, so kann es zu einem Sättigungseffekt kommen, wenn alle Nutzsignalpfade mit einem positiven Storm beaufschlagt werden.

Zum Aufprägen unterschiedlicher Ströme bzw. Signale auf die Nutzsignalpfade weist die Detektionsvorrichtung ganz besonders bevorzugt je Nutzsignalpfad eine Stromaufbringungseinheit auf. Eine Stromaufbringungseinheit umfasst bevorzugt eine Stromquelle. Besonders bevorzugt und wie bereits erwähnt umfasst die Stromaufbringungseinheit aber einen Pull-up- bzw. Pull-down-Widerstand, welcher die Spannungen entlang der Nutzsignalpfade herauf bzw. hinunter regelt und somit indirekt die aufgeprägten Ströme auf dem jeweiligen Nutzsignalpfad beeinflusst.

Dabei ist die Stromaufbringungseinheit bevorzugt so ausgebildet, dass sie auf eine Anzahl von Nutzsignalpfaden einen positiven Strom und auf eine Anzahl von Nutzsignalpfaden einen negativen Strom aufprägt. Besonders bevorzugt entspricht die Anzahl an Nutzsignalpfaden, auf welche ein positiver Strom aufgeprägt wird, der Anzahl an Nutzsignalpfaden, auf welche ein negativer Strom aufgeprägt wird. Dadurch kann abwechselnd ein Nutzsignalpfad mit einem positiven Strom beaufschlagt werden und ein Nutzsignalpfad mit einem negativen Strom beaufschlagt werden. Im Falle eines oder mehrerer Kabeldefekte ergibt sich dadurch ein Gesamtstrom von:

$$I_D = \sum_i I_{Pi} + \sum_j I_{Nj}$$

$I_D$ : Defektstrom

$I_{Pi}$ : positiver Strom im defekten Nutzsignalpfad i

$I_{Nj}$ : negativer Strom im defekten Nutzsignalpfad j

Besonders bevorzugt sind auch die Summen aller Defektströme, also alle möglichen Kombinationen der aufgeprägten Ströme einzigartig bzw. individuell. Kann nun ein Strom auf der Schirmung gemessen werden, kann schnell und einfach aufgrund des Betrags des Signalanteils bzw. der Signalanteile bestimmt werden, welcher bzw. welche Nutzsignalpfad(e) einen Signalpfaddefekt aufweist.

Gerade bei aufwändigen Kabelbäumen von bis zu 200 Leitungen, wie dies beispielsweise bei intrakardialen EKGs, wie z. B. in Angiographie-Anwendungen, der Fall ist, ermöglicht dies eine gezielte Detektion der defekten Leitung. Somit kann anstatt eines kompletten hochkomplexen Kabels im Wert von bis zu 1000€ eine Einzelleitung im Wert von 10-20€ ausgetauscht werden.

Um zu überprüfen, ob die aufgeprägten Signale auf den einzelnen Nutzsignalpfaden im Messbereich liegen, umfasst die Fehlerdetektionseinheit vorzugsweise nicht eine gesamte Vergleicheinheit für alle Nutzsignalpfade, sondern weist für jeden Nutzsignalpfad eine Vergleicheinheit auf. Bevorzugt umfassen die Vergleicheinheiten, je einen AD-Wandler, besonders bevorzugt umfassen sie aber auch je einen Komparator.

Aufgrund etwaiger weiterer Toleranzen und parasitärer Ströme im Spannungsmesssystem unterscheiden sich die aufgeprägten Signale je Nutzsignalpfad bevorzugt um mindestens 5 nA und/oder maximal 20 nA. Ganz besonders bevorzugt um ca. 10 nA.

Um bei einem Signalpfaddefekt leicht den Nutzsignalpfad herauszufinden, welcher den Defekt aufweist, ist die Stromaufbringungseinheit alternativ oder zusätzlich vorzugsweise so ausgebildet, dass sie die aufgeprägten Signale je Nutzsignalpfad einzeln schalten kann. So kann nach Detektion eines Signalpfaddefekts z.B. schrittweise jeweils die Aufprägung eines Signals auf einen Nutzsignalpfad deaktiviert werden. Erzeugt eine Deaktivierung eines Signals auf einem Nutzsignalpfad keine Veränderung des Gesamtsignals auf dem Störsignalpfad, so weist dieser Nutzsignalpfad einen Kabeldefekt auf.

Bevorzugt umfasst die Detektionsvorrichtung des Weiteren zumindest einen ersten Störsignalpfad zur Messung eines ersten Störsignals. Bei der Messung von bioelektrischen Signalen treten wie oben beschrieben z. B. häufig Gleichtaktstörsignale auf.

Der erste Störsignalpfad ist vorzugsweise über eine Elektrode mit dem Patienten verbunden. Daher kann vorzugsweise der dritte Nutzsignalpfad ganz oder zumindest teilweise mit dem ersten Störsignalpfad, welcher nachfolgend näher erläutert wird, zusammenfallen bzw. diesem zumindest abschnittsweise entsprechen. Z. B. können für den dritten Nutzsignalpfad und den ersten Störsignalpfad dieselbe Elektrode und dasselbe Kabel verwendet werden. Es ist also dann nicht einmal erforderlich, dass das Bedienpersonal zusätzliche Elektroden für die erfindungsgemäße Signalpfadprüfung am Patienten anlegt oder sonstige spezielle Maßnahmen durchführt.

Der erste und/oder der dritte Störsignalpfad weist bevorzugt eine Strommesseinheit auf. Diese Strommesseinheit umfasst vorzugsweise einen Strommesswiderstand, bei welchem es sich bevorzugt um einen Shunt-Widerstand handelt, und eine zu diesem parallel geschaltete Spannungsmesseinrichtung.

Der Strommesswiderstand kann dabei zwischen die dritte Elektrode und die Treiberschaltung der Signalmessschaltung, d.h. den Right-Leg-Drive, geschaltet werden.

Es ist bevorzugt, dass der Shunt-Widerstand mindestens einen Widerstandswert von 10 k$\Omega$ und maximal einen Widerstandswert von 1000 k$\Omega$ aufweist.

Bei der Spannungsmesseinrichtung handelt es sich vorzugsweise auch um einen Differenzverstärker. Der Störsignalpfad weist am Ausgang der Spannungsmesseinrichtung eine Störsignalerfassungseinheit auf, um das gemessene Störsignal weiter verarbeiten zu können. Die Störsignalerfassungseinheit umfasst z. B. einen A/D-Wandler und eine Einheit, um das digitale Signal weiter zu verarbeiten.

Beispielsweise kann innerhalb des ersten Störsignals oder eines daraus resultierenden bzw. weiterverarbeitenden Signals, z. B. im Zeit- und/oder Frequenzbereich, nach typischen Merkmalen des bioelektrischen Signals gesucht werden, beispielweise in einem EKG-Signal nach den typischen EKG-Zacken.

Vorzugsweise umfasst eine erfindungsgemäße Detektionsvorrichtung zumindest eine erste Vergleicheinheit, welche überprüft, ob das Signal eines Nutzsignalpfads innerhalb eines Messbereichs liegt.

Hierzu kann vorzugsweise ein definierter Messbereich bzw. ein Schwellwert gewählt werden, ab dem davon ausgegangen wird, dass das Signal auf dem Nutzsignalpfad fließt.

Im Regelfall, also bei intakten Nutzsignalpfaden bzw. Kabeln, sind die Eingangsimpedanzen der Messleitungen der Kabel eines EKG-Messsystems hoch.

Wenn die Elektroden am Patienten angebracht sind, erfolgt bevorzugt eine Überprüfung mittels zumindest der ersten Vergleichseinheit, ob das Signal, das auf den ersten Nutzsignalpfad aufgeprägt wurde, innerhalb eines Messbereichs liegt. Zeitgleich oder aber auch danach wird zumindest ein erstes Störsignal auf zumindest dem ersten Störsignalpfad gemessen.

Ist der Nutzsignalpfad wider Erwarten nicht mit dem Patienten verbunden, da sich beispielsweise die Elektrode vom Patienten gelöst hat, so ist der Stromkreis nicht geschlossen bzw. die vom aufgeprägten Signal zu überwindende Impedanz ist wesentlich größer.

Daher geht die Spannung, welche durch die Stromaufbringungseinheit beispielsweise an der Elektrode des ersten Nutzsignalpfads bewirkt wurde, in die Sättigung. Der Strom kann dabei beispielsweise indirekt über einen Widerstand als Spannung detektiert werden. Das aufgeprägte Signal liegt somit außerhalb des definierten Messbereichs.

Diese Überprüfung bzw. Messung wird mittels der Vergleichseinheit durchgeführt. Die Vergleichseinheit kann also überprüfen, ob der Nutzsignalpfad mit einem Patienten verbunden ist oder nicht. Sie allein gibt aber noch keinen Aufschluss darüber, ob ein Signalpfaddefekt eines mit einem Patienten verbundenen Nutzsignalpfads vorliegt.

Bei intakten und angeschlossenen Kabeln fließt also der aufgeprägte Strom über den ersten Störsignalpfad ab, im Falle eines Kabeldefekts jedoch über die Schirmung. Wenn also, wie oben beschrieben, festgestellt wurde, dass die Kabel korrekt am Patienten angebracht sind, lässt sich ein Kabeldefekt nicht nur über den zusätzlich auf der Schirmung fließenden Strom feststellen. Zudem kann der Kabeldefekt auch mittels des auf dem ersten Störsignalpfad fehlenden Stroms bzw. Signal festgestellt werden. Ohne wesentlich höheren Aufwand können somit zwei Methoden zum Erkennen von Kabeldefekten bereitgestellt und ggf. kombiniert werden.

Die Detektionsvorrichtung weist bevorzugt einen zweiten Störsignalpfad zur Messung eines zweiten Störsignals auf.

Dieser Störsignalpfad kann auf unterschiedliche Weise aufgebaut sein. Er kann derart aufgebaut sein, dass keine bioelektrischen Signale eingekoppelt werden. Es können aber vorzugsweise Störsignale eingekoppelt werden, die auch auf dem ersten Störsignalpfad auftreten, wie beispielsweise die oben beschriebenen Gleichtaktstörsignale. Der zweite Störsignalpfad kann bevorzugt zur Referenzmessung für das Störsignal auf dem ersten Störsignalpfad dienen.

Der zweite Störsignalpfad muss hierbei kein Signalmesskabel umfassen, sondern kann einer kapazitiven Messung bzw. Kopplung gegen Erde entsprechen.

Bevorzugt verläuft der zweite Störsignalpfad zwischen einem Bezugspotential des Spannungsmesssystems bzw. des EKG-Messsystems und einem externen Bezugspotential, z. B. dem Erdpotential. Diese elektrische Kopplung verläuft vorzugsweise über eine kapazitive Kopplung. Indem der zweite Störsignalpfad nur über das gemeinsame Bezugspotential mit dem Spannungsmesssystem gekoppelt ist, ist das zweite Störsignal auf dem zweiten Störsignalpfad weitgehend unabhängig von den Eingangsimpedanzen der verwendeten Kabel in den Nutzsignalpfaden. Der zweite Störsignalpfad kann daher nicht als Rückpfad für die auf Nutzsignalpfade aufgeprägten Signale dienen. Zudem wird das Störsignal aufgrund des Aufbaus des zweiten Störsignalpfads daher weitgehend von Gleichtaktstörsignalen bestimmt.

Zur Realisierung der kapazitiven Kopplung weist der zweite Störsignalpfad vorzugsweise eine mit dem Bezugspotential des Spannungsmesssystems elektrisch verbundene Leiterfläche zwischen dem Spannungsmesssystem und dem Erdpotential auf. Hierbei entspricht die Leiterfläche einer Koppelkapazität. Die Leiterfläche kann z. B. durch eine Metallplatte oder Folie realisiert werden.

Auch der zweite Störsignalpfad kann eine Strommesseinheit aufweisen. Die Strommesseinheit kann hierbei vorzugsweise zwischen das Bezugspotential des Spannungsmesssystems und der kapazitiven Anbindung an das externe Bezugspotential der Leiterfläche geschaltet sein. Des Weiteren kann auch diese Strommesseinheit vorzugsweise einen Strommesswiderstand und eine zu diesem parallel geschaltete Spannungsmesseinrichtung aufweisen. Bei dem Strommesswiderstand handelt es sich vorzugsweise um einen Shunt-Widerstand und bei der Spannungsmesseinrichtung bevorzugt um einen Differenzverstärker.

Der zweite Störsignalpfad kann z. B. am Ausgang der Spannungsmesseinrichtung eine Störsignalerfassungseinheit aufweisen.

Die Detektionsvorrichtung weist bevorzugt eine Störsignalauswerteeinheit als Analyseeinheit auf, die an der Schirmung ein drittes Störsignal misst. Die Schirmung dient somit als dritter Störsignalpfad. Wie oben bereits beschrieben, ist dies besonders vorteilhaft, da die Schirmung einen elektrischen Leiter darstellt, der die gleiche Länge wie die Nutzsignalpfade hat. Elektromagnetische Felder der Umgebung, die zumeist von dem Gleichtaktanteil der Netzfrequenz dominiert werden, koppeln daher ähnlich in die Schirmung wie in den Nutzsignalpfad ein. Dadurch wird eine besonders zweckdienliche und vereinfachte Analyse der Störsignale, insbesondere in Bezug zu den tatsächlich zu messenden bioelektrischen Signalen, möglich.

Sofern zumindest zwei Störsignalpfade genutzt werden, kann die Störsignalauswerteeinheit bevorzugt mit allen Störsignalpfaden verbunden sein. Die Störsignalauswerteeinheit ist dann bevorzugt ausgebildet, um ein Kombinationssignal, des ersten Störsignals, des zweiten Störsignals und/oder des dritten Störsignals zu bilden.

So kann ein Signalpfaddefekt bzw. Kabeldefekt insbesondere nachgewiesen werden, wenn die auf die Nutzsignalpfade aufgeprägten Signale im Differenzsignal mit der Schirmung bzw. dem dritten Störsignalpfad detektiert werden können. Das Differenzsignal setzt sich z. B. also aus dem ersten und/oder zweiten Störsignal und dem dritten Störsignal mittels gewichteter Addition bzw. Subtraktion zusammen. Die Gewichtung kann dabei in geeigneter Weise angepasst werden.

Wird ein Strom als Wechselstrom auf einen Nutzsignalpfad aufgeprägt, kann es sein, dass dieser Strom dem Strom auf dem ersten Störsignalpfad sehr ähnlich ist. Wird nun ein Differenzsignal aus dem ersten und zweiten Störsignal gebildet, lassen sich die vom Nutzsignalpfad übergekoppelten Ströme leichter detektieren.

Das Kombinationssignal kann beispielsweise aber auch ein Verhältnis des ersten und zweiten Störsignals umfassen.

Bevorzugt umfasst die Störsignalauswerteeinheit eine Störsignalunterdrückungseinheit, die so ausgebildet ist, dass sie Störsignalanteile der Nutzsignale auf Basis zumindest eines der ermittelten Störsignale reduziert. Dies kann beispielsweise erfolgen, indem die gemessenen Nutzsignale nachbearbeitet werden, wie später noch näher beschrieben wird. Alternativ oder zusätzlich kann aber auch während der Erfassung der Messsignale eine aktive Gegenregelung über einen entsprechenden Treiber (RLD) am Patienten vorgenommen werden.

Bevorzugt beträgt der Bereich der Netzfrequenz für die Ermittlung des Störsignals ±2%, besonders bevorzugt ±1%, einer Sollnetzfrequenz. Dies gibt im Wesentlichen den Rahmen an der bei einem funktionierenden Stromnetz nicht verlassen werden sollte. Daher ist es vorteilhaft, die Analyse auf diesen Bereich zu beschränken.

Dabei kann die tatsächliche Netzfrequenz vom Sollwert, der Sollnetzfrequenz, abweichen. Die Sollnetzfrequenz beträgt z. B. 60 Hz in Nord-, Mittel- und Teilen Südamerikas sowie in Japan, Taiwan, Philippinen etc. oder z. B. 50 Hz in den anderen Teilen der Welt.

Die Störsignalunterdrückung erfolgt bevorzugt, indem zum Beispiel mit der Information über dieses Signal ein frequenzbasierter Filter eingestellt wird, den vorteilhafterweise eine schmalere Bandbreite aufweisen kann als bisher im Stand der Technik üblich. Der Einfluss der Störsignale und des Filterprozesses auf die Nutzsignale bzw. die bioelektrischen Signale kann vorteilhafterweise verringert werden.

Alternativ kann bevorzugt auch eine Phasenregelschleife (PLL, engl. "phase-locked loop") zur Unterdrückung der Störsignale eingesetzt werden, die das erfindungsgemäß ermittelte Störsignal als Generator-Eingangssignal erhält. Ein PLL ist eine elektronische Schaltungsanordnung, die die Phasenlage und damit zusammenhängend die Frequenz eines veränderbaren Oszillators über einen geschlossenen Regelkreis so beeinflusst, dass die Phasenabweichung zwischen einem äußeren Referenzsignal (dem Störsignal) und einem daraus abgeleiteten Signal möglichst konstant ist. Dem PLL wird somit vorteilhafterweise bereits sehr genaue Information über die Frequenz des Störsignals bereitgestellt, sodass er nur noch die gegebenenfalls verschobene Phase bzw. unterschiedliche Amplitude der Störung in den Nutzsignalen bzw. den bioelektrischen Signalen ermitteln muss. Dadurch konvergiert die PLL vorteilhafterweise schneller gegen die finale Qualität des störunterdrückten Signals.

Vorzugsweise weist die Detektionsvorrichtung eine Ausgabeeinheit auf, die an den Ausgang der Störsignalauswerteeinheit angeschlossen ist und/oder extern, z. B. über eine Funkübertragung arbeitet. Die Ausgabeeinheit dient dazu, einen detektierten Signalpfaddefekt auszugeben bzw. gleich zu signalisieren. Diese Ausgabe oder Signalisierung kann dabei vor Ort z. B. optisch, akustisch erfolgen. Zudem kann die Signalisierung per Funk z. B. an einen Servicetechniker gesendet werden. Eine weitere Ausgabeform kann als Protokollierung, z. B. gemeinsam mit den Messdaten erfolgen. Besonders bevorzugt erfolgt die Protokollierung zeitlich korreliert zum Messsignal bzw. den zu messenden bioelektrischen Signalen. So kann z.B. bei einer nur zeitweise auftretenden Störung wie bei einem Wackelkontakt dokumentiert werden, welche Messwerte verwendbar sind und welche nicht.

Insbesondere wenn die Detektionsvorrichtung in dem Spannungsmesssystem integriert ist, ist die Ausgabeeinheit bevorzugt in einer Benutzerschnittstelle des Spannungsmesssystems inkludiert. Dadurch kann beispielsweise das Bedienpersonal auf der Benutzerschnittstelle, z.B. einen Monitor, gleichzeitig die bioelektrischen Signale überprüfen und einen Kabeldefekt detektieren.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:

FIG 1 schematisch eine Ausführungsbeispiel eines differentiellen Spannungsmesssystems inklusive einer möglichen Positionierung der elektrischen Anschlüsse bzw. Kontakte eines an einem Patienten,

FIG 2 schematisch ein differentielles Spannungsmesssystem mit einer Störsignalmesseinrichtung gemäß einem Ausführungsbeispiel der Erfindung,

FIG 3 ein schematisches Blockdiagramm eines Ausführungsbeispiels einer Störsignalauswerteeinheit für ein erfindungsgemäßes differentielles Spannungsmesssystem,

FIG 4 ein Flussdiagramm, welches ein Verfahren zur Erzeugung eines störreduzierten biologischen Messsignals gemäß einem Ausführungsbeispiel der Erfindung veranschaulicht,

FIG 5 schematisch ein differentielles Spannungsmesssystem mit einer Störsignalmesseinrichtung gemäß einem zweiten Ausführungsbeispiel der Erfindung,

FIG 6 ein schematisches Blockdiagramm eines Ausführungsbeispiels einer Störsignalauswerteeinheit,

FIG 7 ein schematischen Ablaufdiagramm eines Ausführungsbeispiels eines erfindungsgemäßen Verfahrens zur Unterdrückung von Störsignalen in einem differentiellen Spannungsmesssystem.

In den Figuren wird exemplarisch jeweils von einem EKG-Messsystem 1 als differentielles Spannungsmesssystem 1 ausgegangen, um bioelektrische Signale S(k), hier EKG-Signale S(k), zu messen. Die Erfindung ist aber nicht hierauf beschränkt.

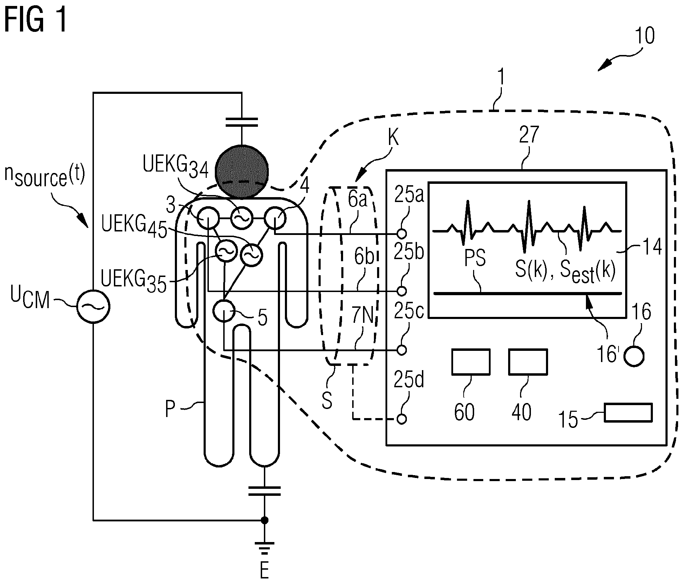

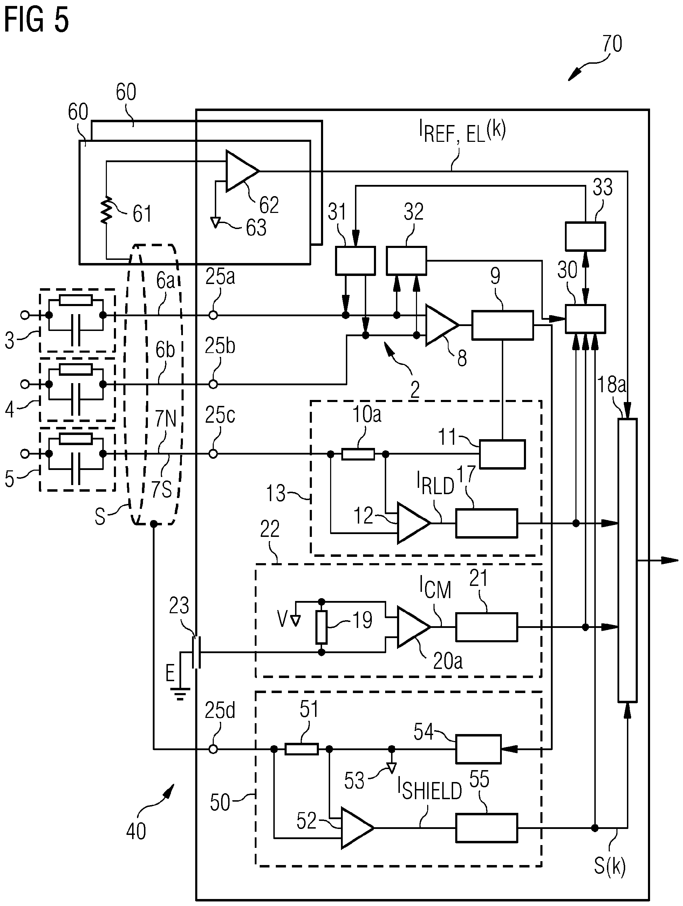

FIG 1 zeigt beispielhaft eine Anordnung 10 mit einem erfindungsgemäßen EKG-Messsystem 1 an einem Patienten P. Das EKG-Messsystem 1 umfasst ein EKG-Gerät 27 mit seinen elektrischen Anschlüssen und daran über Kabel K angeschlossene Elektroden 3, 4, 5, um an dem Patienten P EKG-Signale S(k) zu messen. Dieses EKG-Messsystem 1 ist mit Hilfe der Erfindung in der Lage, Störsignale, welche auf die Elektroden 3, 4, 5 eingekoppelt werden (wie er beispielsweise in FIG 5 zu sehen ist), zu unterdrücken.

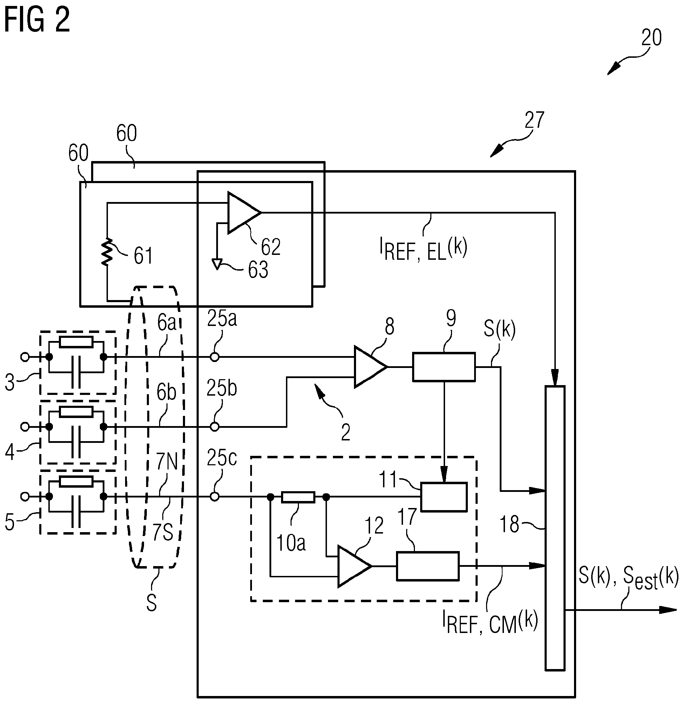

Um die EKG-Signale S(k) zu messen, werden mindestens eine erste Elektrode 3 und eine zweite Elektrode 4 benötigt, die auf dem Patienten P angebracht sind. Durch Signalmesskabel K sind die Elektroden 3, 4 über Anschlüsse 25a, 25b meist Steckverbindungen 25a, 25b mit dem EKG-Gerät 27 verbunden. Die erste Elektrode 3 und die zweite Elektrode 4 einschließlich der Signalmesskabel K bilden dabei einen Teil einer Signalerfassungseinheit 9 (siehe FIG 2) mit der die EKG-Signale S(k) erfasst werden können.

Eine dritte Elektrode 5 dient als Referenzelektrode, um einen Potentialausgleich zwischen dem Patienten P und dem EKG-Gerät 27 zu schaffen. Dies wird später noch genauer erläutert. Klassisch wird diese dritte Elektrode 5 am rechten Bein des Patienten angebracht (weshalb wie oben erwähnt dieser Anschluss oft auch als "Right-Leg-Drive" bzw. "RLD" bezeichnet wird). Sie kann aber, wie hier auch, an einer anderen Stelle positioniert werden. Darüber hinaus können über weitere Anschlüsse, welche in den Figuren nicht dargestellt sind, am EKG-Gerät 27 noch eine Vielzahl weiterer Kontakte für weitere Ableitungen (Potentialmessungen) am Patienten P angebracht und für die Bildung von geeigneten Signalen genutzt werden.

Zwischen den einzelnen Elektroden 3, 4, 5 bilden sich die Spannungspotentiale $UEKG_{34}$, $UEKG_{45}$ und $UEKG_{35}$ die zur Messung der EKG-Signale S(k) dienen.

Die direkt gemessenen EKG-Signale S(k) und/oder weiter verarbeitete bioelektrische Signale $S_{est}(k)$ werden auf einer Benutzerschnittstelle 14 des EKG-Geräts 27 angezeigt.

Der Patient P ist bei der EKG-Messung zumindest kapazitiv mit dem Erdpotential E gekoppelt (in FIG 1 durch eine Kopplung am Kopf und dem rechten Bein schematisch dargestellt). Er unterliegt jedoch einer Störquelle $U_{cm}$, beispielsweise einem durch die Stromversorgung mit 50 Hz Wechselstrom entstehenden elektrischen Feld, und dem daraus resultierenden über den Patienten P vorliegenden und sich mit der Zeit t ständig verändernden Störsignal $n_{source}(t)$, welches durch die relativ empfindliche Messung zwangsläufig miterfasst wird. Durch diese Störquelle $U_{cm}$ werden in der Regel Störsignale über den Patienten P auf die Messleitungen in den Signalmesskabeln K eingekoppelt, auf die später noch verwiesen wird.

Die Signalmesskabel K, welche von der ersten Elektrode 3 und der zweiten Elektrode 4 zum EKG-Gerät 27 führen, sind hierbei ein Teil der Nutzsignalpfade 6a, 6b. Das Signalmesskabel K, welches von der Elektrode 5 zum EKG-Gerät 27 führt, entspricht hierbei einem Teil eines dritten Nutzsignalpfads 7N. Der dritte Nutzsignalpfad 7N überträgt Störsignale der Störquelle $U_{cm}$, welche über den Patienten P und die Elektroden eingekoppelt wurden.

Die Kabel K weisen eine Schirmung S auf, die hier schematisch als ein alle Nutzsignalpfade 6a, 6b, 7N umgebender gestrichelter Zylinder dargestellt ist. Die Schirmung muss aber nicht alle Kabel K gemeinsam umgeben, sondern die Kabel K können auch separat geschirmt sein. Die Anschlüsse 25a, 25b, 25c weisen jedoch bevorzugt jeweils integriert einen Pol für die Schirmung auf. Diese Pole werden dann auf einen gemeinsamen Schirmungsanschluss 25d zusammengeführt. Die Schirmung S ist dabei z. B. als eine den Leiter des jeweiligen Kabels K umgebende Metallfolie ausgebildet, die jedoch von dem Leiter isoliert ist.

Um auf den ersten und/oder den zweiten Nutzsignalpfad eingekoppelte Störsignale zu erkennen, weist das EKG-Gerät eine Störfeld-Messschaltung 60 auf, welche ein aufgrund in die Elektroden einkoppelnder elektrischer Felder auftretende Potentialänderung auf der Schirmung S misst.

Um Kabeldefekte D zu erkennen, kann das EKG-Messsystem 1 optional eine Detektionsvorrichtung 40, welche anhand von FIG 5 noch genauer erläutert wird, aufweisen. Mit Hilfe dieser Detektionsvorrichtung 40 werden die Kabel K auf Kabeldefekte D überprüft. Das durch die Detektionsvorrichtung 40 generierte Prüfsignal PS, welches einen Kabeldefekt D signalisiert, kann wie in FIG 1 gezeigt, durch eine Ausgabeeinheit 16' auf der Benutzerschnittstelle 14 des EKG-Geräts 27 angezeigt und dargestellt werden. Hierdurch können auf der Benutzerschnittstelle 14 nicht nur die EKG-Signale S(k), sondern zeitgleich auch die Kabel K auf einen eventuellen Kabeldefekt D überwacht werden.

Die Ausgabeeinheit 16 muss aber nicht zwingend in der Benutzerschnittstelle 14 integriert sein. Die Signalisierung kann z. B. auch über eine Signalleuchte, beispielsweise in Form einer LED (Leuchtdiode) oder dergleichen, realisiert werden, die einen Defekt signalisiert. Sie kann aber zusätzlich oder alternativ auch akustisch z. B. über einen Warnton erfolgen. Eine weitere Variante ist auch eine externe Übermittlung z. B. über Funk an einen Servicetechniker oder zur Ausgabe in einem Messprotokoll, um so einen Kabeldefekt D anzuzeigen bzw. zu protokollieren. Zudem kann das EKG-Gerät 27, wie in FIG 1 gezeigt, eine externe Schnittstelle 15 aufweisen, um beispielweise einen Anschluss für einen Drucker, eine Speichereinrichtung und/oder sogar ein Netzwerk bereit zu stellen.

In FIG 2 ist sehr grob schematisch ein Ausführungsbeispiel eines EKG-Messsystems 20 gemäß einem Ausführungsbeispiel der Erfindung detaillierter in einem Blockschaltbild veranschaulicht.

Das EKG-Messsystem 20 umfasst eine Signalmessschaltung 2, welche zur Messung der bioelektrischen Signale S(k) dient.

Die Signalmessschaltung 2 hat hier, wie oben bereits erwähnt, drei Nutzsignalpfade 6a, 6b, 7N. Die Nutzsignalpfade sind, wie im Zusammenhang mit FIG 1 beschrieben, über die Elektroden 3, 4, 5, die Kabel K und die Steckverbindung 25a, 25b, 25c vom Patienten P mit dem EKG-Gerät 27 elektrisch verbunden. Die Elektroden 3, 4, 5 sind hier vereinfacht als ein RC-Glied dargestellt und veranschaulichen die Impedanz-Werte der Nutzsignalpfade 6a, 6b, 7N.

Die erste Elektrode 3 und die zweite Elektrode 4 stehen mit dem Patienten P in Kontakt. Aufgrund einer Potentialdifferenz zwischen den Ableitungsstellen, an denen die Elektroden 3, 4 am Patienten befestigt sind, wird ein Nutzsignal, z. B. ein "Herzstrom" von den Elektroden 3, 4 zu einer Verstärkerschaltung 8, z. B. einem Operationsverstärker, übertragen. Die Verstärkerschaltung 8 umfasst zwei Eingänge und ist über diese mit der ersten Elektrode 3 und zweiten Elektrode 4 elektrisch verbunden. Das Ausgangssignal der Verstärkerschaltung 8 wird an eine Signalerfassungseinheit 9 übermittelt, welche das von der Verstärkerschaltung 8 verstärkte Nutzsignal erfasst. Der erste Nutzsignalpfad 6a verläuft hierbei vom Kontakt der ersten Elektrode 3 zu dem Patienten P über die erste Elektrode 3 bis zum Eingang der Verstärkerschaltung 8. Der zweite Nutzsignalpfad 6b verläuft von dem Kontakt der zweiten Elektrode 4 zu dem Patienten P über die zweite Elektrode 4 zum Eingang der Verstärkerschaltung 8.

Die in Zusammenhang mit FIG 1 beschriebene dritte Elektrode 5 ist Teil des ersten Störsignalpfades 7S. Sie ist über das Kabel K mit einem Strommesswiderstand 10a, im folgenden Shunt-Widerstand genannt, elektrisch verbunden. Der Shunt-Widerstand 10a ist zudem mit einer Treiberschaltung 11 elektrisch verbunden, welche, wie bereits erläutert, auch als Right-Leg-Drive bezeichnet wird. Die Treiberschaltung 11 ist so aufgebaut, dass über die Elektrode 5 ein Referenzpotential an den Patienten angelegt wird, welches den Gleichtaktspannungen mit EKG-Anteilen entspricht. Beispielsweise kann dieses Referenzpotential in bekannter Weise auf einen inversen, verstärkten Mittelwert der Messleitungen gesetzt werden. Dadurch kann das Referenzpotential auf die Gleichtaktspannung festgelegt werden.

Das in der RLD-Schaltung erzeugte Referenzsignal $I_{REF,CM}$ wird außerdem an eine Auswerteeinheit 18 übermittelt, welche auch das mit Hilfe der ersten und zweiten Elektrode 3, 4 erfasste Messsignal S(k) empfängt und ein entstörtes Signal $S_{est}(k)$ ausgibt.

Teil der in FIG 2 gezeigten Messschaltung 20 ist auch eine für jede Elektrode 3, 4 ausgebildete Störfeld-Messschaltung 60, welche eine aufgrund in die Elektroden einkoppelnder elektrischer Felder auftretende Potentialänderung auf der Schirmung S misst. Für die Messung weist die Störfeld-Messschaltung 60 jeweils einen Shunt-Widerstand 61 und einen Messverstärker 62 auf. Das von der Messschaltung 60 erzeugte Elektroden-Referenzstörsignal $I_{Ref,El}(k)$ wird ebenfalls an die Auswerteinheit 18 übermittelt.

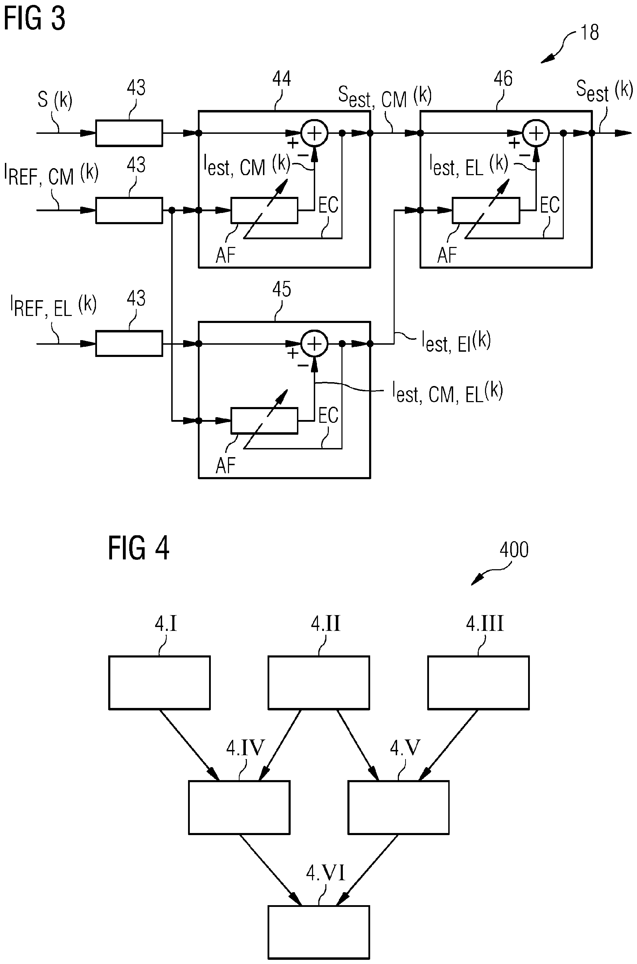

In FIG 3 ist ein eine schematische Darstellung einer Auswerteeinheit 18 gemäß einem Ausführungsbeispiel der Erfindung veranschaulicht. Die Auswerteeinheit 18 weist jeweils Eingänge bzw. digitale Filter 43 auf, welche die von der Signalmessschaltung 2, der Treiberschaltung 11 und der Messschaltung 21 erzeugten Signale S(k), $I_{REF,CM}$, $I_{Ref,El}(k)$ erfassen und filtern. Die genannten Filter 43 sind üblicherweise als Bandpassfilter bzw. Sperrfilter ausgebildet.

Dabei trägt das Messsignal S(k) Signalstöranteile $I_{EKG,CM}(k)$, welche durch Einkopplung elektrischer Felder auf den Körper erzeugt werden, und Signalstöranteile $I_{EKG,EL}(k)$, welche durch Einkopplung elektrischer Felder auf die Elektroden 3, 4 erzeugt wurden.

$$S(k) = I_{EKG}(k) + I_{EKG,CM}(k) + I_{EKG,EL}(k).$$

Zusammen mit dem Referenzsignal $I_{REF,CM}$ der Störungen auf den Körper wird mit einem ersten adaptiven Filter 44 ein störreduziertes Signal $S_{est,CM}(k)$ erzeugt, welches weniger Signalanteile enthält, die auf den Körper eingekoppelt sind. Bei dem Filterprozess wird als Störsignal ein Korrektursignal $I_{est,CM}(k)$ geschätzt und mit dem eingehenden Signal S (k) zu dem störreduzierten Signal Sest,CM(k) kombiniert.

Allerdings enthält dieses störreduzierte Signal $S_{est,CM}(k)$ auch bereits weniger Signalanteile der Störung, welche auf die Elektrode eingekoppelt ist. Aus diesem Grund lässt sich das Elektroden-Referenz-Störsignal $I_{Ref,El}(k)$ nicht mehr als Referenz für dieses bereits teilweise entstörte Signal $S_{est,CM}(k)$ zur weiteren Filterung nutzen. Aus diesem Grund wird zusätzlich mit Hilfe eines zweiten adaptiven Filters 45 auf Basis des Elektroden-Referenz-Störsignals $I_{Ref,El}(k)$ und des auf den Körper des Patienten eingekoppelten Referenz-Störsignals $I_{REF,CM}(k)$ ein angepasstes Elektrodenstörsignal $I_{est,El}(k)$ erzeugt. Bei dem Filterprozess wird als Störsignal ein Korrektursignal $I_{eSt,CM,EL}(k)$ geschätzt und mit dem eingehenden Referenz-Störsignal $I_{Ref,El}(k)$ zu dem angepassten Elektrodenstörsignal $I_{est,El}(k)$ kombiniert.

Das angepasste Elektrodenstörsignal $I_{est,El}(k)$ wird schließlich zur adaptiven Filterung in einem dritten adaptiven Filter 46 des bereits teilweise störreduzierten Signals $S_{est,CM}(k)$ genutzt, um ein fast störfreies Endsignal $S_{est}(k)$ zu erhalten. Bei dem dritten Filterprozess wird als Störsignal ein Korrektursignal $I_{est,EL}(k)$ geschätzt und mit dem eingehenden störreduzierten Signal $S_{est,CM}(k)$ zu dem fast störfreien Endsignal $S_{est}(k)$ kombiniert.

In FIG 4 ist ein Flussdiagramm 400 gezeigt, welches ein Verfahren zur Erzeugung eines störreduzierten biologischen Messsignals gemäß einem Ausführungsbeispiel der Erfindung veranschaulicht. Bei dem Schritt 4.I erfolgt zunächst ein Erfassen und Bandpassfiltern eines Messsignals $S(k)$. Parallel dazu wird bei dem Schritt 4.II auch ein Signalstöranteil $I_{REF,CM}(k)$, welcher durch Einkopplung elektrischer Felder auf den Körper erzeugt wird, gemessen. Weiter parallel werden bei dem Schritt 4.III Signalstöranteile $I_{REF,EL}(k)$ gemessen, welche durch Einkopplung elektrischer Felder auf die Elektroden 3, 4 erzeugt wurden. Bei dem Schritt 4.IV erfolgt ein erster adaptiver Filterprozess, bei dem die beiden bei dem Schritt 4.I und 4.II erfassten Signale $S(k)$ und $I_{REF,CM}(k)$ durch eine gewichtete Subtraktion miteinander kombiniert werden. Die Gewichtung kann dabei nach Bedarf, also z. B. nach der Stärke der jeweiligen Einkopplungen bzw. Störfelder variiert werden. Durch diesen Vorgang wird ein weitgehend von Signalstöranteilen, welche durch Einkopplung elektrischer Felder auf den Körper erzeugt werden, befreites Signal $S_{est,CM}(k)$ erzeugt. Bei dem Schritt 4.V wird weiterhin auf Basis der beiden bei dem Schritt 4.II und 4.III erzeugten Referenz-Störsignale $I_{REF,CM}(k)$, $I_{REF,EL}(k)$ ein angepasstes Elektroden-Referenz-Störsignal $I_{est,El}(k)$ erzeugt, welches bereits von SignalAnteilen befreit ist, welche zwar eine Störung betreffen, die auf eine Elektrode 3, 4 des Nutzsignalpfads eingekoppelt wird, aber bereits in dem bei dem Schritt 4.II erzeugten Referenzstörsignal $I_{REF,CM}(k)$ enthalten ist. Auf diese Weise wird ein angepasstes Elektroden-Referenz-Störsignal $I_{est,El}(k)$ erzeugt. Das angepasste Elektroden-Referenz-Störsignal $I_{est,El}(k)$ wird bei dem Schritt 4.VI in einem dritten adaptiven Filtervorgang mit dem bei dem Schritt 4.IV erzeugten Signal

$S_{est,CM}(k)$ durch eine gewichtete Subtraktion kombiniert. Dabei wird als Ergebnis ein weitgehend entstörtes Signal $S_{est}(k)$ erzeugt, welches weder nennenswerte Störungen enthält, die durch Einkopplung elektrischer Felder auf den Körper entstanden sind, noch nennenswerte Störungen enthält, die durch Einkopplung elektrischer Felder auf die Elektroden 3, 4 entstanden sind.

In FIG 5 ist ein zweites Ausführungsbeispiel eines EKG-Geräts 70 eines EKG-Messsystems 1 detaillierter in einem Blockschaltbild veranschaulicht.

Zusätzlich zu den in FIG 2 gezeigten Bauelementen weist das in FIG 5 gezeigte EKG-Gerät 70 eine Detektionseinrichtung 40 mit einer Stromaufbringungseinheit 31 auf. Diese kann zum einen ein erstes Signal, hier ein sich im Nanoampere-Bereich befindlicher erster Strom $I_{E1}$, auf den ersten Nutzsignalpfad 6a aufprägen. Zum anderen kann sie ein zweites Signal $I_{E2}$, hier ein sich im Nanoampere-Bereich befindlicher zweiter Strom $I_{E2}$, auf den zweiten Nutzsignalpfad 6b aufprägen. Zudem ist der zweite Strom $I_{E2}$ hier ein um 10 nA erhöhter Strom im Vergleich zum ersten Strom $I_{E1}$.

Mittels einer Stromaufbringungskontrolleinheit 33, die mit der Stromaufbringungseinheit 31 und einer Defektanalyseeinheit 30, die später noch beschrieben wird, kommuniziert, werden Ströme geregelt.

Bei intakten Signalmesskabeln besteht für den ersten Strom $I_{E1}$ und den zweiten Strom $I_{E2}$ außer dem Störsignalpfad 7S kein weiterer niederohmiger Rückpfad auf das gemeinsame Erdpotential.

Das heißt, dass sich auf dem Störsignalpfad 7S nicht nur die Störsignale $I_{CM}$ befinden, sondern auch der auf dem ersten Störsignalpfad 6a aufgeprägte Strom $I_{E1}$ und der auf dem zweiten Nutzsignalpfad 6b aufgeprägte Strom $I_{E2}$.

Es ergibt sich also bei intakten Nutzsignalpfaden 6a, 6b folgendes Störsignal $I_{RLD}$ auf dem ersten Störsignalpfad 7S:

$$I_{RLD} = I_{CM} + I_{E1} + I_{E2}.$$

Die Stromaufbringungseinheit 31 wurde hier exemplarisch nur einmal dargestellt, sie kann aber beispielsweise mittels einer ersten Stromquelle, die den ersten Strom $I_{E1}$ auf den ersten Nutzsignalpfad 6a aufprägt und einer zweiten Stromquelle, die den zweiten Strom $I_{E2}$ auf den zweiten Nutzsignalpfad 6b aufprägt, realisiert werden.

Die Spannungen, welche durch die Stromaufbringungseinheit 31 an der ersten Elektrode 3 und der zweiten Elektrode 4 erzeugt wurden, befinden sich regelmäßig höchstens im Millivolt-Bereich. Denn die aufgeprägten Ströme fließen im Nanoampere-Bereich durch eine Impedanz ab, die in einem Bereich von ca. 50 kOhm bis 2 MOhm liegen kann. Diese Impedanz ist somit jedenfalls geringer als die der Nutzsignalpfade. Ist der erste Nutzsignalpfad 6a und/oder der zweite Nutzsignalpfad 6b jedoch nicht mit dem Patienten P elektrisch verbunden, so ist der Stromkreis nicht geschlossen bzw. die vom aufgeprägten Strom zu überwindende Impedanz wesentlich größer. Dadurch geht die durch die Stromquellen erzeugte Spannung an der Elektrode, die keinen Kontakt zu dem Patienten P aufweist, in Sättigung. Um dies zu überprüfen, weist die Detektionsvorrichtung 40 eine Vergleichseinheit 32 auf. Die Vergleichseinheit 32 wurde hier zur Anschauung nur als ein Block dargestellt. Es liegt hier jedoch für den ersten Nutzsignalpfad 6a und für den zweiten Nutzsignalpfad 6b bevorzugt jeweils eine Vergleichseinheit vor. Die Vergleichseinheiten 32 umfassen hier Komparatoren. Liegen die ermittelten Ströme auf dem ersten Nutzsignalpfad 6a und auf dem zweiten Nutzsignalpfad 6b innerhalb eines vorgegebenen Messbereichs, so sind die erste Elektrode 3 und die zweite Elektrode 4 mit einem Patienten elektrisch verbunden und die Komparatoren 32 melden zwei verbundene Elektroden.

Wird beispielsweise nur eine verbundene Elektrode gemeldet, so kann ein Anwender des EKG-Geräts 70 sofort die Elektroden überprüfen und eventuell neu anbringen.

Wie zuvor beschrieben, werden die Elektroden über Signalmesskabel K an das EKG-Gerät 70 angeschlossen. Um die Anwendung des EKG-Messsystems 1 am Patienten P möglichst einfach zu gestalten, sollen die Kabel K schlank, leicht und zugleich geschirmt sein. Diese Merkmalskombination führen jedoch oft zu Kabeldefekten D. Diese Kabeldefekte D können nach einer Biegung oder Torsion der Kabel K entstehen. Hierbei kann es zu einer irreversiblen Auswölbung der Messleitungen kommen, welche die Leitungs-Isolation durchbricht. Durch die Durchbrechung der Leitungs-Isolation kann ein Kontakt zwischen den Messleitungen und der Schirmung S entstehen. Durch diesen Kontakt kommt es zu einer Reduzierung der Eingangsimpedanz und zu einer Verstärkung von Störungen.

Durch die Reduzierung der Eingangsimpedanz des Kabels K bei einem Kabeldefekt D, kommt es zu einem weiteren niederohmigen Rückflusspfad für den jeweiligen aufgeprägten Strom über die Schirmung S. Liegt beispielsweise ein Kabeldefekt D des ersten Nutzsignalpfads 6a vor, so fließt der Strom $I_{E1}$ über die Schirmung S ab und erhöht somit nicht mehr den Strom $I_{RLD}$ auf dem Störsignalpfad 7S. Der Komparator 32 erkennt diesen fehlerhaft abgeflossenen Strom aber nicht und gibt weiterhin an, dass die Elektrode 3 mit dem Patienten P elektrisch verbunden ist.

Um nun diesen Kabeldefekt D detektieren zu können, weist die Detektionsvorrichtung 40 drei Störsignalpfade 7S, 22, 50 auf.

Die Nutzsignalpfade 6a, 6b umfassen die erste Elektrode 3 und die zweite Elektrode 4, die Kabel K, die Stromaufbringungseinheit 31, die Vergleichseinheit 32 sowie die weitere geräteinterne Leitung (mit der Verstärkerschaltung 8) bis zur Signalerfassungseinheit 9 hat hier ebenfalls eine Doppelfunktion. Sie gehören nämlich zum einen zur Signalmessschaltung 2, um bioelektrische Signale S(k) zu messen. Zum anderen gehören sie zur Detektionsvorrichtung 40, um zu überprüfen, ob oder gegebenenfalls wie viele Elektroden der entsprechenden Nutzsignalpfade 6a, 6b mit einem Patienten verbunden bzw. defekt sind.

Das von der Störsignalauswerteeinheit 13 ausgegebene Signal $I_{RLD}$ wird in einer Defektanalyseeinheit 30 zusammen mit den Daten der Vergleichseinheit 32 analysiert. Melden die Vergleichseinheiten 32, dass alle Elektroden verbunden sind und die Störsignalauswerteeinheit 13 gibt einen Strom $I_{RLD}$ aus, welcher die Störsignale $I_{RLD}$ sowie den ersten aufgeprägten Strom $I_{E1}$ und den zweiten aufgeprägten Strom $I_{E2}$ umfasst, so detektiert die Defektanalyseeinheit 30, dass alle Elektroden mit dem Patienten P verbunden sind und kein Kabeldefekt D vorliegt.

Im Defektfall, z. B. des Nutzsignalpfades 6a, fließt der Strom $I_{E1}$ über die Schirmung S ab. Zur Messung dieses Defektstromes weist der dritte Störsignalpfad 50 eine Strommesseinheit 51, 52 auf.

Für die dritte Strommesseinheit 51, 52 wird ein zwischen das interne Bezugspotential 53, das das gleiche Bezugspotential wie V sein kann, und dem Anschluss 25d der Schirmung S geschalteter dritter Shunt-Widerstand 51 als Strommesswiderstand verwendet. Zudem wird eine zu diesem parallel geschaltete dritte Spannungsmesseinrichtung 52 eingesetzt. Die dritte Spannungsmesseinrichtung 52 kann dabei wieder durch einen Verstärker, z. B. durch einen PGA, realisiert werden. Die Spannungsmesseinrichtung 52 ist außerdem mit einer dritten Störsignalerfassungseinheit 55 verbunden, die z. B. als A/D-Wandler ausgebildet ist und die gemessenen Signale $I_{SHIELD}$ digitalisiert und ggf. weiterverarbeitet.

Zudem ist auf dem dritten Störsignalpfad 50 ein Kabeltreiber 54 angeordnet, der mit der Signalerfassungseinheit 9 verbunden ist. Dadurch kann ein Referenzpotential an die Schirmung S angelegt werden, welches komplementär zu den Gleichtaktspannungen ist. Dadurch können die Störfelder der Umgebung bzw. der Netzspannung angenähert werden.

Die Detektionsvorrichtung 40 muss aber nicht, wie z. B. in FIG 5 gezeigt, in dem EKG-Messsystem integriert sein. Sie kann auch in ein bereits bestehendes EKG-Messsystem über beispielsweise Steckverbindungen, eingebaut werden oder auch vor- oder zwischengeschaltet sein. Durch eine solche Nachrüstung ist es möglich, auch mit einem bereits bestehenden EKG-Messsystem Kabeldefekte D zu detektieren.

Um die EKG-Signale S(k) bzw. $S_{est}(k)$ sowie eventuelle Kabeldefekte D mittels eines Prüfsignals PS (siehe FIG 1) auf der Benutzerschnittstelle 14 parallel anzuzeigen, ist diese an die Signalerfassungseinheit 9 der Signalmessschaltung 2 und an die Defektanalyseeinheit 30 der Detektionsvorrichtung 40 angeschlossen. Dies ist in FIG 1 grob schematisch veranschaulicht. Die Benutzerschnittstelle 14 ist daher in FIG 1 mit einer entsprechenden Ausgabeeinheit 16' gezeigt.

Die oben beschriebene weitere Ausgabeeinheit 16 zur beispielsweise optischen und/oder akustischen Signalisierung eines Kabeldefekts D kann ebenfalls mit einem Ausgang der Störsignalauswerteeinheit 18a und der Defektanalyseeinheit 30 gekoppelt werden.

Zudem ist das differentielle Spannungsmesssystem 1, wie schon erwähnt, mit einer externen Schnittstelle 15 beispielsweise für ein Netzwerk, einen Drucker und/oder einen Speicher etc. ausgestattet, die z. B. mit der Signalerfassungseinheit 9 der Signalmessschaltung 2 und/oder Defektanalyseeinheit 30 signaltechnisch verbunden sein kann.

Die Detektionsvorrichtung 40 weist drei Störsignalpfade 7S, 22, 50 auf. Der erste Störsignalpfad 7S umfasst, wie bei dem Ausführungsbeispiel in FIG 2, die dritte Elektrode 5, welche mit ihrem Eingang an einen Patienten P angeschlossen ist, und verläuft bis zu dem Shunt-Widerstand 10a, der mit dem Ausgang der Elektrode 5 elektrisch verbunden ist. Die über dem Shunt-Widerstand 10a abfallende Spannung wird auch hier von der parallel zum Shunt-Widerstand 10a geschalteten ersten Spannungsmesseinrichtung 12 gemessen. Das dadurch gemessene Störsignal $I_{RLD}$ wird anschließend von einer an den Ausgang der ersten Spannungsmesseinrichtung 12 geschalteten ersten Störsignalerfassungseinheit 17 digitalisiert, weiterverarbeitet und erfasst.

Die Detektionsvorrichtung 40 umfasst ferner einen zweiten Störsignalpfad 22 mit einer Strommesseinheit 19, 20a. Mit dieser zweiten Strommesseinheit 19, 20a wird der von einem internen Bezugspotential V des EKG-Geräts 70 über eine kapazitive Kopplung zu einem externen festen Bezugspotential E, dem Erdpotential E, fließende Strom gemessen. Bei diesem zweiten gemessenen Störsignal $I_{CM}$ handelt es sich vor allem wieder um Gleichtaktstörsignale. Die kapazitive Kopplung zwischen dem EKG-Gerät 70 und dem Erdpotential E ist ohnehin immer vorhanden. Um einen definierten Störsignalpfad 22 für dieses Störsignal $I_{CM}$ zur Verfügung zu stellen, an dem das Störsignal $I_{CM}$ gut gemessen werden kann, wird eine großflächigere Leiterfläche 23, z. B. in Form einer Metallplatte oder einer Folie, mit dem internen Bezugspotential V des EKG-Geräts 70 verbunden, welche eine "Kondensatorfläche" gegen das Erdpotential E bildet. Die zweite Strommesseinheit 19, 20a ist in diesem zweiten Störsignalpfad 22 zwischen dem internen Bezugspotential V und der Leiterfläche 23 geschaltet.

Für die zweite Strommesseinheit 19, 20a, wird zur Strommessung auf dem zweiten Störsignalpfad 22 ein zwischen internes Bezugspotential V und Leiterfläche 23 geschalteter Strommesswiderstand 19, im weiteren zweiter Shunt-Widerstand genannt, verwendet und eine zu diesem parallel geschaltete zweite Spannungsmesseinrichtung 20a. Die zweite Spannungsmesseinrichtung 20a kann dabei wieder durch einen Verstärker, z. B. durch einen PGA, realisiert werden.

Das gemessene zweite Störsignal $I_{CM}$ wird durch eine an den Ausgang der Spannungsmesseinrichtung 20a geschaltete Störsignalerfassungseinheit 21 erfasst, z. B. durch einen A/D-Wandler digitalisiert und gegebenenfalls weiterverarbeitet.

Die drei Störsignalerfassungseinheiten 17, 21, 55 der jeweiligen Störsignalpfade 7S, 22, 50 sind mit einer - hier vorzugsweise digital arbeitenden - Störsignalauswerteeinheit 18 sowie mit der Defektanalyseeinheit 30 verbunden.

Die Störsignalauswerteeinheit 18a ist eingerichtet, um das erste Störsignal $I_{RLD}$, das zweite Störsignal $I_{CM}$ und das dritte Störsignal $I_{SHIELD}$ zu verarbeiten. Dadurch können die Gleichtaktstörungen auf dem dritten Störsignalpfad 50 von den dort bei defekten Kabeln K bzw. Messleitungen K auftretenden übergekoppelten Strömen $I_{E1}$, $I_{E2}$ getrennt bzw. unterschieden werden. Infolgedessen kann ein Kabeldefekt D leichter nachgewiesen werden. Zur Auswertung der Störsignale $I_{RLD}$, $I_{CM}$, $I_{SHIELD}$, die ja hier digital vorliegen, kann die Störsignalauswerteeinheit 18 wieder durch eine Recheneinrichtung mit geeigneter Software realisiert werden und/oder beispielsweise auch durch einen oder mehrere ASIC.

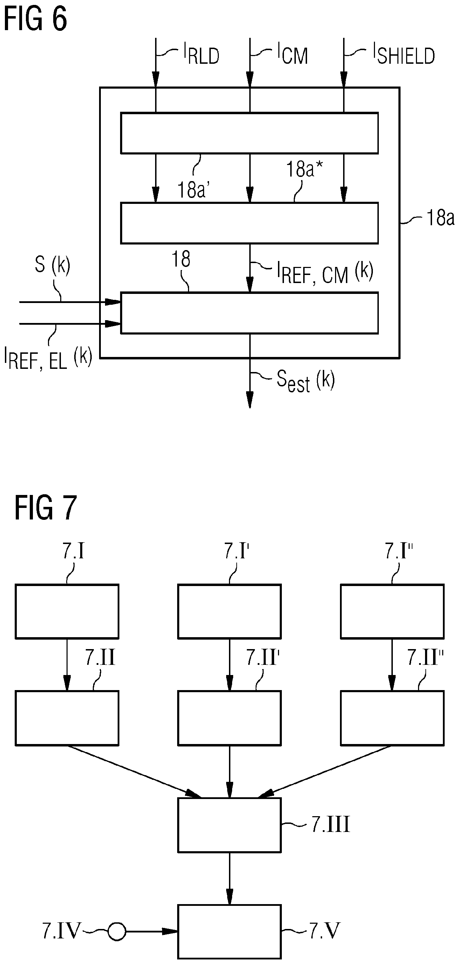

Vorzugsweise kann die Störsignalauswerteeinheit 18a so ausgebildet sein, dass aus den Störsignalen $I_{RLD}$, $I_{CM}$, $I_{Shield}$ ein Ausgangssignal $I_{REF,CM}(k)$ erzeugt wird, wie anhand von FIG 6 detaillierter erläutert wird.

Teil der in FIG 5 gezeigten Messschaltung 70 ist auch eine für jede Elektrode 3, 4 ausgebildete Störfeld-Messschaltung 60, welche eine aufgrund in die Elektroden einkoppelnder elektrischer Felder auftretende Potentialänderung auf der Schirmung S misst. Für die Messung weist die Störfeld-Messschaltung 60 jeweils einen Shunt-Widerstand 61 und einen Messverstärker 62 auf. Das von der Störfeld-Messschaltung 60 erzeugte Elektroden-Referenzstörsignal $I_{Ref,El}(k)$ wird ebenfalls an die Auswerteinheit 18a übermittelt.

Neben der verbesserten Störsignalkompensation lässt sich also mit Hilfe der in FIG 5 im Detail veranschaulichten Messschaltung 70 ein Kabeldefekt D in einem EKG-System 1 sofort und eindeutig detektieren. Hierfür muss kein gesondertes Testverfahren von einem geschulten Servicetechniker durchgeführt werden. Die Überprüfung der Kabel K verläuft simultan zur EKG-Messung und Defekte D können von jedem Bedienpersonal des EKG-Geräts schnell und einfach detektiert werden. Werden zudem unterschiedliche Ströme auf die Nutzsignalpfade aufgeprägt, ist es auch möglich, zu bestimmen, welcher Nutzsignalpfad einen Signalpfaddefekt aufweist.

Liegen die überprüften Ströme, die auf die Nutzsignalpfade 6a, 6b aufgeprägt worden sind, im Messbereich und es wurden beispielsweise zwei verbundene Elektroden detektiert und die Strommessung auf dem ersten Störsignalpfad misst ebenfalls die Ströme die über zwei Elektroden auf den ersten Störsignalpfad eingekoppelt wurden, so liegen beide Elektroden an und es liegt kein Signalpfaddefekt vor. Dies kann zusätzlich oder alternativ auch festgestellt werden, wenn auf der Schirmung keiner der aufgeprägten Ströme fließt.

Liegen die überprüften Ströme, die auf die Nutzsignalpfade 6a, 6b aufgeprägt worden sind, im Messbereich und es wurden beispielsweise zwei verbundene Elektroden detektiert, aber die Strommessung auf dem ersten Störsignalpfad misst beispielsweise nur einen Strom, der über eine Elektrode auf den Störsignalpfad eingekoppelt wurde, so

liegt ein Signalpaddefekt eines Nutzsignalpfads vor. Dann fließt aber der Strom des defekten Nutzsignalpfads über die Schirmung ab, und kann dort alternativ oder zusätzlich detektiert werden.

FIG 6 und FIG 7 werden im Folgenden gemeinsam beschrieben. Dabei zeigt FIG 6 ein Ausführungsbeispiel einer Störsignalauswerteeinheit 18a. Eine solche Störsignalauswerteeinheit 18a ermittelt sowohl auftretende Störungen als auch ein von Störungen befreites Messsignal $S_{est}(k)$. In FIG 7 ist ein Ausführungsbeispiel eines erfindungsgemäßen Verfahrens zur Detektion von Störungen und zur Ermittlung eines von Störungen befreiten Messsignals $S_{est}(k)$ schematisch als Blockdiagramm dargestellt. Die Störsignalauswerteeinheit 18a empfängt in den Schritten 7.I, 7.I', 7.1" als Eingangssignale jeweils die in den Störsignalerfassungseinheiten 17, 21, 55 zuvor erfassten Störsignale $I_{RLD}$, $I_{CM}$, $I_{SHIELD}$.

Aus diesen bloßen Messsignalen werden in einer Störsignalermittlungseinheit 18a' der Störsignalauswerteeinheit 18a Störsignale mit einer Frequenz im Bereich einer Netzfrequenz extrahiert. Dies erfolgt im Schritt 7.II für das erste Störsignal $I_{RLD}$, im Schritt 7.II' für das zweite Störsignal $I_{CM}$ und im Schritt 7.II" für das dritte Störsignal $I_{SHIELD}$ jeweils beispielsweise mittels einer Frequenzanalyse.

Im Schritt 7.III werden die derart auf eine Frequenz präzisierten Signale in einer Kombinationseinheit 18a* der Störsignalauswerteeinheit 18a zu einem Kombinationssignal zusammengefasst, welches dem bereits im Zusammenhang mit FIG 2 bis FIG 5 beschriebenen Referenz-Störsignal $I_{Ref,CM}(k)$ entspricht. Dieser Vorgang kann beispielsweise mittels einer gewichteten Addition bzw. Subtraktion der Signale erfolgen. Die Gewichtung kann dabei nach Bedarf, also z. B. nach der Stärke der jeweiligen Einkopplungen bzw. Störfelder variiert werden.

In einem Schritt 7.IV werden bioelektrische Messsignale $S(k)$ von der Störsignalauswerteeinheit 18a empfangen, die zuvor von der Signalmessschaltung 2 ermittelt wurden.

Die Störsignalauswerteeinheit 18a umfasst ferner eine Störsignalunterdrückungseinheit 18. Diese dient dazu, die Störsignalanteile auf Basis des ermittelten Referenz-Störsignals $I_{Ref,CM}(k)$ zu verringern. Zusätzlich erhält die Störsignalunterdrückungseinheit 18 auch ein von der Störfeld-Messschaltung 60 erzeugtes Referenz-Störsignal $I_{Ref,El}(k)$, welches ebenfalls zur Reduktion der Störeffekte genutzt wird. Die genaue Funktion der Störsignalunterdrückungseinheit 18 ist in FIG 3 und FIG 4 illustriert. Die Erzeugung eines störungsarmen Signals $S_{est}(k)$ erfolgt im Schritt 7.V auf die in FIG 4 veranschaulichte Art und Weise.

Alternativ können aber auch eine Vielzahl von weiteren Algorithmen verwendet werden, wie beispielsweise eine Mustererkennung oder ein Kalman-Filter, um die Referenz-Störsignale $I_{Ref,CM}(k)$, $I_{Ref,El}(k)$ auszuwerten.

Es wird abschließend noch einmal darauf hingewiesen, dass es sich bei den vorhergehend detailliert beschriebenen Vorrichtungen und Verfahren lediglich um Ausführungsbeispiele handelt, welche vom Fachmann in verschiedenster Weise modifiziert werden können, ohne den Bereich der Erfindung zu verlassen. So kann es sich bei dem differentiellen Spannungsmesssystem nicht nur um ein EKG-Gerät handeln, sondern auch um andere medizinische Geräte mit denen bioelektrische Signale erfasst werden, wie beispielsweise EEGs, EMGs usw. Weiterhin schließt die Verwendung der unbestimmten Artikel "ein" bzw. "eine" nicht ausschließt, dass die betreffenden Merkmale auch mehrfach vorhanden sein können. Ebenso schließt der Begriff "Einheit" nicht aus, dass diese aus mehreren Komponenten besteht, die gegebenenfalls auch räumlich verteilt sein können.

**Patentansprüche**

1. Störsignalmesseinrichtung (60) in einem differentiellen Spannungsmesssystem (1) mit einer Signalmessschaltung zur Messung von bioelektrischen Signalen (S(k)) mit einer Anzahl von Nutzsignalpfaden (6a; 6b) mit jeweils einer Sensorelektrode (3, 4), aufweisend:

   - eine zusätzliche Sensorleitung pro Sensorelektrode (3, 4), welche jeweils mit einer Masseverbindung einer Zuleitung einer Sensorelektrode (3, 4) elektrisch verbunden ist,
   - eine Messverstärkerschaltung (62) je Sensorelektrode (3, 4), welche jeweils über einen elektrischen Widerstand (61) mit der zusätzlichen Sensorleitung verbunden ist und dazu eingerichtet ist, eine auf der Sensorleitung auftretende elektrische Potentialänderung zu erfassen und daraus ein Elektroden-Referenz-Störsignal ($I_{Ref,EL}$) zu ermitteln,

   **dadurch gekennzeichnet, dass**
   die Messverstärkerschaltung (62) an die Schirmung (S) einer Zuleitung der Sensorelektrode (3, 4) elektrisch angeschlossen ist.

2. Störsignalmesseinrichtung nach Anspruch 1, wobei die Messverstärkerschaltung (62) eine programmierbare Verstärkerschaltung aufweist.

3. Störsignalkompensationseinrichtung (60, 18, 18a), aufweisend

- eine Störsignalmesseinrichtung (60) nach einem der Ansprüche 1 oder 2,
- eine Auswerteeinheit (18, 18a) mit

- einer ersten adaptiven Filtereinheit (44), welche dazu eingerichtet ist, ein erstes störreduziertes Messsignal ($S_{est,CM}(k)$) auf Basis eines Messsignals ($S(k)$) und eines Referenz-Gleichtaktstörsignals ($I_{Ref,CM}(k)$) zu erzeugen,
- einer zweiten adaptiven Filtereinheit (45), welche dazu eingerichtet ist, ein um Gleichtaktstörungen reduziertes angepasstes Referenz-Elektroden-Störsignal ($I_{est,El}(k)$) auf Basis des von der Störsignalmesseinrichtung (60) ermittelten Elektroden-Referenz-Störsignals ($I_{Ref,EL}$) und des Referenz-Gleichtaktstörsignals ($I_{Ref,CM}(k)$) zu erzeugen,

- einer dritten adaptiven Filtereinheit (46), welche der ersten (44) und der zweiten Filtereinheit (45) nachgeschaltet ist und dazu eingerichtet ist, ein zweites störreduziertes Messsignal ($S_{est}(k)$ auf Basis des ersten störreduzierten Messsignals ($S_{est,CM}(k)$) und des angepassten Referenz-Elektroden-Störsignals ($I_{est,El}(k)$) zu ermitteln.

4. Differentielles Spannungsmesssystem (1), aufweisend:

- mindestens eine erste Elektrode (3) und eine zweite Elektrode (4) zur Messung von bioelektrischen Messsignalen ($S(k)$),
- mindestens eine dritte Elektrode (5) für einen Potentialausgleich zwischen einem Messobjekt (P) und dem differentielle Spannungsmesssystem (1),
- eine Messeinrichtung (27, 70) mit

- einer Signalmessschaltung (2) zur Messung der bioelektrischen Signale ($S(k)$)
- einer Referenzsignaleinheit (13), welche ein Referenz-Gleichtaktstörsignal ($I_{Ref,CM}(k)$) erfasst,
- einer Störsignalmesseinrichtung (60) nach einem der Ansprüche 1 oder 2.

5. Differentielles Spannungsmesssystem nach Anspruch 4, wobei die Messeinrichtung (70) eine Detektionsvorrichtung (40) zur Detektion von Störungen auf Signalpfaden (6a, 6b) in dem differentiellen Spannungsmesssystem (1), aufweist, welche folgende Komponente umfasst:

- zumindest eine Analyseeinheit (18, 30), welche mit der Schirmung (S) verbunden ist und ausgebildet ist, um eine Störung (D, $U_{CM}$) in einem Nutzsignalpfad (6a, 6b) des Spannungsmesssystems (10) mittels eines im Störfall an einer Schirmung (S) der Signalpfade gemessenen Signals ($I_{E1}$, $I_{E2}$, $I_{SHIELD}$) zu detektieren.

6. Differentielles Spannungsmesssystem nach Anspruch 4 oder 5, wobei die Detektionsvorrichtung aufweist:

- zumindest eine Stromaufbringungseinheit (31), wobei die Stromaufbringungseinheit (31) so ausgebildet ist, dass sie ein Signal ($I_{E1}$, $I_{E2}$) auf einen ersten Nutzsignalpfad (6a, 6b) aufprägt,
- eine Defektanalyseeinheit (30) als Analyseeinheit, die einen Signalpfaddefekt in einem Nutzsignalpfad (6a, 6b) des Spannungsmesssystems (1) mittels des zuvor aufgeprägten, im Störfall an der Schirmung (S) gemessenen Signals ($I_{E1}$, $I_{E2}$) detektiert.

7. Differentielles Spannungsmesssystem nach Anspruch 6, wobei die Stromaufbringungseinheit (31) jeweils verschiedene Signale ($I_{E1}$, $I_{E2}$) auf unterschiedliche Nutzsignalpfade (6a, 6b) aufprägt.

8. Differentielles Spannungsmesssystem nach Anspruch 7, umfassend

- zumindest eine erste Vergleichseinheit (32), welche überprüft, ob das Signal eines Nutzsignalpfads (6a, 6b) innerhalb eines Messbereichs liegt.

9. Differentielles Spannungsmesssystem nach Anspruch 7, umfassend einen zweiten Störsignalpfad (22) zur Messung eines zweiten Störsignals ($I_{CM}$).

10. Differentielles Spannungsmesssystem nach einem der vorstehenden Ansprüche 4 bis 9 mit einer Störsignalauswerteeinheit (18a) als Analyseeinheit, die an der Schirmung (S) ein drittes Störsignal ($I_{SHIELD}$) misst.

**11.** Differentielles Spannungsmesssystem nach Anspruch 10, wobei die Störsignalauswerteeinheit (18a) so ausgebildet ist, um ein Kombinationssignal ($I_{Ref,CM}(k)$) des ersten Störsignals ($I_{RLD}$), des zweiten Störsignals ($I_{CM}$) und/oder des dritten Störsignals ($I_{SHIELD}$) zu bilden.

**12.** Verfahren zur Erzeugung eines störreduzierten biologischen Messsignals ($S_{est}(k)$) mittels einer Störsignalkompensationseinrichtung nach Anspruch 3, aufweisend die Schritte:

- Erfassen eines möglicherweise störbehafteten Messsignals (S(k),
- Erfassen eines Elektroden-Referenz-Störsignals ($I_{Ref,EL}$) über eine Messverstärkerschaltung (62) je Sensorelektrode (3, 4), welche jeweils über einen elektrischen Widerstand (61) mit der zusätzlichen Sensorleitung verbunden ist und dazu eingerichtet ist, eine auf der Sensorleitung auftretende elektrische Potentialänderung zu erfassen und daraus ein Elektroden-Referenz-Störsignal ($I_{Ref,EL}$) zu ermitteln,
- Erfassen eines Referenz-Gleichtakt-Störsignals ($I_{Ref,EL}$),
- Erzeugen eines ersten störreduzierten Messsignals ($S_{est,CM}(k)$) auf Basis eines Messsignals (S(k)) und des Referenz-Gleichtaktstörsignals ($I_{Ref,CM}(k)$),
- Erzeugen eines um Gleichtaktstörungen reduzierten angepassten Referenz-Elektroden-Störsignals ($I_{est,El}(k)$) auf Basis des von der Störsignalmesseinrichtung (60) ermittelten Elektroden-Referenz-Störsignals ($I_{Ref,EL}$) und des Referenz-Gleichtaktstörsignals ($I_{Ref,CM}(k)$),
- Erzeugen eines zweiten störreduzierten Messsignals ($S_{est}(k)$ auf Basis des ersten störreduzierten Messsignals ($S_{est,CM}(k)$) und des angepassten Referenz-Elektroden-Störsignals ($I_{est,El}(k)$).

**13.** Computerprogrammprodukt mit einem Computerprogramm, welches direkt in eine Speichereinrichtung eines Spannungsmesssystems (1) nach Anspruch 4 ladbar ist, mit Programmabschnitten, um alle Schritte des Verfahrens nach Anspruch 12 auszuführen, wenn das Computerprogramm in dem Spannungsmesssystem (1) ausgeführt wird.

**14.** Computerlesbares Medium, auf welchem das Computerprogrammprodukt nach Anspruch 13 gespeichert ist.

**Claims**

**1.** Interference signal measuring facility (60) in a differential voltage measuring system (1) with a signal measuring circuit for measuring bioelectric signals (S(k)) with a number of useful signal paths (6a; 6b) each with a sensor electrode (3, 4) having:

- an additional sensor lead for each sensor electrode (3, 4), each of which is electrically connected to a ground connection of a supply lead of a sensor electrode (3, 4),
- a measuring amplifier circuit (62) for each sensor electrode (3, 4) each of which is connected to the additional sensor lead via an electrical resistor (61) and is configured to detect a change in electric potential occurring on the sensor lead and to determine an electrode reference interference signal ($I_{Ref,EL}$) therefrom
**characterised in that**
the measuring amplifier circuit (62) is electrically connected to the shield (S) of a supply lead of the sensor electrode (3, 4).

**2.** Interference signal measuring facility according to claim 1, wherein the measuring amplifier circuit (62) has a programmable amplifier circuit.

**3.** Interference signal compensation facility (60, 18, 18a) having

- an interference signal measuring facility (60) according to one of claims 1 or 2,
- an evaluation unit (18, 18a) with

- a first adaptive filter unit (44) which is configured to generate a first interference-reduced measurement signal ($S_{est,CM}(k)$) based on a measurement signal (S(k)) and a reference common-mode interference signal ($I_{Ref,CM}(k)$),

- a second adaptive filter unit (45), which is configured to generate an adapted reference electrode interference

signal ($I_{est,EI}(k)$) reduced by common-mode interferences based on the electrode reference interference signal ($I_{Ref,EL}$) determined by the interference signal measuring facility (60) and the reference common-mode interference signal ($I_{Ref,CM}(k)$,

- a third adaptive filter unit (46) arranged downstream of the first (44) and second filter unit (45) configured to determine a second interference-reduced measurement signal ($S_{est}(k)$ based on the first interference-reduced measurement signal ($S_{est,CM}(k)$) and the adapted reference electrode interference signal ($I_{est,EI}(k)$).

4. Differential voltage measuring system (1) having:

- at least one first electrode (3) and one second electrode (4) for measuring bioelectric measurement signals ($S(k)$),
- at least one third electrode (5) for potential equalization between a measurement object (P) and the differential voltage measuring system (1),
- a measuring facility (27, 70) with

- a signal measuring circuit (2) for measuring the bioelectric signals ($S(k)$)
- a reference-signal unit (13) which detects a reference common-mode interference signal ($I_{Ref,CM}(k)$),
- an interference signal measuring facility (60) according to one of claims 1 or 2.

5. Differential voltage measuring system according to claim 4, wherein the measuring facility (70) has a detection apparatus (40) for detecting interferences on signal paths (6a, 6b) in the differential voltage measuring system (1) which comprises the following components:

- at least one analysis unit (18, 30) connected to the shield (S) and embodied to detect interference (D, $U_{CM}$) in a useful signal path (6a, 6b) of the voltage measuring system (10) by means of a signal ($I_{E1}$, $I_{E2}$, $I_{SHIELD}$) measured in the event of interference on a shield (S) of the signal paths.

6. Differential voltage measuring system according to claim 4 or 5, wherein the detection apparatus has:

- at least one current application unit (31), wherein the current application unit (31) is embodied to impress a signal ($I_{E1}$, $I_{E2}$) on a first useful signal path (6a, 6b),
- a defect analysis unit (30) as an analysis unit which detects a signal path defect in a useful signal path (6a, 6b) of the voltage measuring system (1) by means of the previously impressed signal ($I_{E1}$, $I_{E2}$) measured in the event of interference on the shield (S).

7. Differential voltage measuring system according to claim 6, wherein the current application unit (31) in each case impresses different signals ($I_{E1}$, $I_{E2}$) onto different useful signal paths (6a, 6b).

8. Differential voltage measuring system according to claim 7 including

- at least one first comparison unit (32) which checks whether the signal of a useful signal path (6a, 6b) is within a measuring range.

9. Differential voltage measuring system according to claim 7 including a second interference signal path (22) for measuring a second interference signal ($I_{CM}$).

10. Differential voltage measuring system according to one of the preceding claims 4 to 9 with an interference signal evaluation unit (18a) as an analysis unit which measures a third interference signal ($I_{SHIELD}$) on the shield (S).

11. Differential voltage measuring system according to claim 10, wherein the interference signal evaluation unit (18a) is embodied to form a combination signal ($I_{Ref,CM}(k)$) of the first interference signal ($I_{RLD}$), the second interference signal ($I_{CM}$) and/or the third interference signal ($I_{SHIELD}$).

12. Method for generating an interference-reduced biological measurement signal ($S_{est}(k)$) by means of an interference signal compensation facility according to claim 3, having the steps:

- detecting a possibly interference-afflicted measurement signal ($S(k)$),
- detecting an electrode reference interference signal ($I_{Ref,EL}$) via a measuring amplifier circuit (62) for each sensor electrode (3, 4) each of which is connected to the additional sensor lead via an electrical resistor (61) and is configured to detect a change in electric potential occurring on the sensor lead and to determine an electrode reference interference signal ($I_{Ref,EL}$) therefrom,
- detecting a reference common-mode-interference signal ($I_{Ref,EL}$),
- generating a first interference-reduced measurement signal ($S_{est,CM}(k)$) based on a measurement signal ($S(k)$) and the reference common-mode interference signal ($I_{Ref,CM}(k)$),
- generating an adapted reference electrode interference signal ($I_{est,EI}(k)$) reduced by common-mode interferences based on the electrode reference interference signal ($I_{Ref,EL}$) determined by the interference signal measuring facility (60) and the reference common-mode interference signal ($I_{Ref,CM}(k)$),
- generating a second interference-reduced measurement signal ($S_{est}(k)$) based on the first interference-reduced measurement signal ($S_{est,CM}(k)$) and the adapted reference electrode interference signal ($I_{est,EI}(k)$) .

**13.** Computer program product with a computer program which can be loaded directly into a storage facility of a voltage measuring system (1) according to claim 4, with program sections for executing all the steps of the method according to claim 12 when the computer program is executed in the voltage measuring system (1).

**14.** Computer-readable medium on which the computer program product according to claim 13 is saved.

**Revendications**

**1.** Dispositif (60) de mesure d'un signal parasite dans un système (1) différentiel de mesure de tension comprenant un circuit de mesure du signal pour la mesure de signaux ($S(k)$) bioélectriques ayant un nombre de chemins (6a ; 6b) de signal utile ayant respectivement une électrode (3, 4) de capteur, comportant :

- une ligne supplémentaire de capteur par électrode (3, 4) de capteur, qui est reliée électriquement respectivement à une liaison à la masse d'une ligne d'alimentation d'une électrode (3, 4) de capteur,
- un circuit (62) amplificateur de mesure par électrode (3, 4) de capteur, qui est reliée respectivement par une résistance (61) électrique à la ligne de capteur supplémentaire et qui est agencée pour détecter une variation de potentiel électrique se produisant sur la ligne de capteur et pour en déterminer un signal ($I_{ref,EL}$) parasite de référence d'électrode,

**caractérisé en ce que**
le circuit (62) amplificateur de mesure est raccordé électriquement au blindage (S) d'une ligne d'alimentation de l'électrode (3, 4) de capteur.

**2.** Dispositif de mesure d'un signal parasite suivant la revendication 1, dans lequel le circuit (62) amplificateur de mesure a un circuit amplificateur programmable.

**3.** Dispositif (60, 18, 18a) de compensation d'un signal parasite, comportant

- un dispositif (60) de mesure d'un signal parasite suivant l'une des revendications 1 ou 2,
- une unité (18, 18a) d'analyse comprenant

- une première unité (44) adaptative de filtrage, qui est agencée pour produire un premier signal ($S_{est,CM}(k)$) de mesure réduit en parasite sur la base d'un signal ($S(k)$) de mesure et d'un signal ($I_{Ref,CM}(k)$) parasite en mode commun de référence,
- une deuxième unité (45) adaptative de filtrage, qui est agencée pour produire un signal ($I_{est,E1}(k)$) parasite d'électrode de référence adapté réduit en parasite en mode commun sur la base du signal ($I_{ref,EL}$) parasite de référence d'électrode déterminé par le dispositif (60) de mesure d'un signal parasite et du signal ($I_{Ref,CM}(k)$) parasite en mode commun de référence,
- une troisième unité (46) adaptative de filtrage, qui est montée après la première (44) et la deuxième (45) unités de filtrage et qui est agencée pour déterminer un deuxième signal ($S_{est}(k)$) de mesure réduit en parasite sur la base du premier signal ($S_{est,CM}(k)$) de mesure réduit en parasite et du signal ($I_{est,E1}(k)$) parasite d'électrode de référence adapté.

**4.** Système (1) différentiel de mesure de tension, comportant :

- au moins une première électrode (3) et une deuxième électrode (4) de mesure de signaux (S(k)) bioélectriques de mesure,
- au moins une troisième électrode (5) de compensation de potentiel entre un objet (P) à mesurer et le système (1) différentiel de mesure de tension,
- un dispositif (27, 70) de mesure comprenant

  - un premier circuit (2) de mesure du signal pour la mesure des signaux (S(k)) bioélectriques,
  - une unité (13) de signal de référence, qui détecte un signal ($I_{Ref,CM}$(k)) parasite en mode commun de référence,
  - un dispositif (60) de mesure d'un signal parasite suivant l'une des revendications 1 ou 2.

5. Système différentiel de mesure de tension suivant la revendication 4, dans lequel le dispositif (70) de mesure a un dispositif (40) de détection pour la détection de parasites sur des chemins (6a, 6b) de signal dans le système (1) différentiel de mesure de tension, qui comprend les composants suivants :

   - au moins une unité (18, 30) d'analyse, qui est reliée au blindage (S) et qui est constituée pour détecter un parasite (D, $U_{CM}$) dans un chemin (6a, 6b) de signal utile du système (10) de mesure de tension au moyen d'un signal ($I_{E1}$, $I_{E2}$, $I_{SHIELD}$) mesuré dans le cas d'une perturbation sur un blindage (S) des chemins du signal.

6. Système différentiel de mesure de tension suivant la revendication 4 ou 5, dans lequel le dispositif de détection comporte :

   - au moins une unité (31) d'application d'un courant, l'unité (31) d'application d'un courant étant constituée de manière à appliquer un signal ($I_{E1}$, $I_{E2}$) à un premier chemin (6a, 6b) de signal utile,
   - une unité (30) d'analyse de défaut, en tant qu'unité d'analyse, qui détecte un défaut de chemin de signal dans un chemin (6a, 6b) du signal utile du système (1) de mesure de tension au moyen du signal ($I_{E1}$, $I_{E2}$) appliqué auparavant, mesuré dans le cas d'une perturbation sur le blindage (S).

7. Système différentiel de mesure de tension suivant la revendication 6, dans lequel l'unité (31) d'application d'un courant applique respectivement des signaux ($I_{E1}$, $I_{E2}$) différents à des chemins (6a, 6b) de signal utile différents.

8. Système différentiel de mesure de tension suivant la revendication 7, comprenant

   - au moins une première unité (32) de comparaison, qui contrôle si le signal d'un chemin (6a, 6b) de signal utile est à l'intérieur d'une plage de mesure.

9. Système différentiel de mesure de tension suivant la revendication 7, comprenant un deuxième chemin (22) de signal parasite pour la mesure d'un deuxième signal ($I_{CM}$) parasite.

10. Système différentiel de mesure de tension suivant l'une des revendications 4 à 9 précédentes, comprenant une unité (18a) d'évaluation de signal parasite en tant qu'unité d'analyse, qui mesure un troisième signal ($I_{SHIELD}$) parasite sur le blindage (S) .

11. Système différentiel de mesure de tension suivant la revendication 10, dans lequel l'unité (18a) d'évaluation d'un signal parasite est constituée pour former un signal ($I_{Ref,CM}$(k)) de combinaison du premier signal ($I_{RLD}$) parasite, du deuxième signal ($I_{CM}$) parasite et/ou du troisième signal ($I_{SHIEL}$) parasite.

12. Procédé de production d'un signal ($S_{est}$(k)) de mesure biologique réduit en parasite au moyen d'un dispositif de compensation de signal parasite suivant la revendication 3, comportant les stades :

    - détection d'un signal (S(k)) de mesure entaché éventuellement de parasites,
    - détection d'un signal ($I_{Ref,EL}$) parasite de référence d'électrode par un circuit (62) amplificateur de mesure pour chaque électrode (3, 4) de mesure, qui est reliée respectivement par une résistance (61) électrique à la ligne de capteur supplémentaire et qui est agencée pour détecter une modification de potentiel électrique se produisant sur la ligne de capteur et en déterminer un signal ($I_{Ref,EL}$) parasite de référence d'électrode,
    - détection d'un signal ($I_{Ref,EL}$) parasite en mode commun de référence,
    - production d'un premier signal ($S_{est,CM}$(k)) de mesure réduit en parasite sur la base d'un signal (S(k)) de mesure et du signal ($I_{Ref,CM}$(k)) parasite en mode commun de référence,

- production d'un signal ($I_{est,E1}(k)$) parasite d'électrode de référence adapté réduit en parasite en mode commun sur la base du signal ($I_{Ref,EL}$) parasite de référence d'électrode déterminé par le dispositif (60) de mesure de signal parasite et du signal ($I_{Ref,CM}(k)$) parasite en mode commun de référence,
- production d'un deuxième signal ($S_{est}(k)$) de mesure réduit en parasite sur la base du premier signal ($S_{est,CM}(k)$) de mesure réduit en parasite et du signal ($I_{est,E1}(k)$) parasite d'électrode de référence adapté.

13. Produit de programme d'ordinateur comprenant un programme d'ordinateur, qui peut être chargé directement dans un dispositif de mémoire d'un système (1) de mesure de tension suivant la revendication 4, comportant des parties de programme pour effectuer tous les stades du procédé suivant la revendication 12, lorsque le programme d'ordinateur est réalisé dans le système (1) de mesure de tension.

14. Support, déchiffrable par ordinateur, sur lequel est mis en mémoire le produit de programme d'ordinateur suivant la revendication 13.

FIG 1

FIG 2

## FIG 3

18

S (k) — 43

$I_{REF, CM}$ (k) — 43

$S_{est, CM}$ (k)

44

$I_{est, CM}$ (k)

+
−

EC

AF

46

$S_{est}$ (k)

$I_{est, EL}$ (k)

+
−

EC

AF

$I_{REF, EL}$ (k) — 43

45

$I_{est, EI}$(k)

+
−

$I_{est, CM, EL}$(k)

EC

AF

## FIG 4

400

4.I

4.II

4.III

4.IV

4.V

4.VI

FIG 5

$I_{REF, EL}(k)$

$I_{RLD}$

$I_{CM}$

$I_{SHIELD}$

$S(k)$

## FIG 6

$I_{RLD}$    $I_{CM}$    $I_{SHIELD}$

18a'

18a*

18a

$S(k)$

18

$I_{REF, CM}(k)$

$I_{REF, EL}(k)$

$S_{est}(k)$

## FIG 7

7.I     7.I'     7.I''

7.II     7.II'     7.II''

7.III

7.IV    7.V

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 7993167 B2
- US 2008312523 A1
- DE 102017214862 A1
- EP 3569143 A1
- DE 102015202447 A1